(19) Europäisches Patentamt
European Patent Office
Office européen des brevets

(11) **EP 3 598 999 A1**

(12) **DEMANDE DE BREVET EUROPEEN**

(43) Date de publication:
**29.01.2020 Bulletin 2020/05**

(21) Numéro de dépôt: **19196582.1**

(22) Date de dépôt: **22.07.2016**

(51) Int Cl.:
***A61Q 19/08*** *(2006.01)* ***A61Q 17/04*** *(2006.01)*
***A61K 8/9789*** *(2017.01)* ***A61K 8/37*** *(2006.01)*
***A61K 8/49*** *(2006.01)* ***A61K 36/486*** *(2006.01)*

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA MD**

(30) Priorité: **22.07.2015 FR 1556973**

(62) Numéro(s) de document de la (des) demande(s) initiale(s) en application de l'article 76 CBE:
**16745672.2 / 3 325 103**

(71) Demandeur: **Biosynthis**
**91410 Saint Cyr Sous Dourdan (FR)**

(72) Inventeurs:
- **BERNOUD, Thierry**
  **91410 SAINT CYR SOUS DOURDAN (FR)**
- **PICCIRILLI, Antoine**
  **86000 POITIERS (FR)**
- **MAGNE, Julien**
  **86340 les Roches-Prémarie-Andillé (FR)**

(74) Mandataire: **Pellegri, Michel Pascal Romain**
**Cabinet Tripoz**
**Le Pôle Sud**
**22, rue Seguin**
**69002 Lyon (FR)**

Remarques:
Cette demande a été déposée le 10-09-2019 comme demande divisionnaire de la demande mentionnée sous le code INID 62.

(54) **PROCEDE D'ENRICHISSEMENT D'HUILE DE KARANJA**

(57) L'invention concerne un ingrédient cosmétique, caractérisé en ce qu'il consiste en une solution d'au moins un extrait d'huile de karanja comprenant du pongamol (CAS 484-33-3) et de la karanjine (CAS 521-88-0) dans au moins un solvant choisi dans le groupe des diesters de formule (I) suivante, ou leurs mélanges :

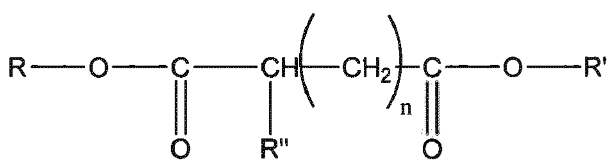

Formule (I)

dans laquelle :
- n est compris entre 0 et 19 ;
- R et R' identiques ou différents sont choisis dans le groupe des alkyls issus d'un alcool linéaire ou ramifié de formule brute CxH2x+2O avant l'estérification, x étant compris entre 1 et 30, préférentiellement entre 1 et 20, préférentiellement entre 1 et 10.
- R" est soit un atome d'hydrogène soit un groupement alkyle en C1-C3.

L'invention concerne également un procédé de précipitation sélective, une formulation cosmétique ainsi que ses utilisations.

EP 3 598 999 A1

Printed by Jouve, 75001 PARIS (FR)

## Description

La présente invention concerne le domaine de l'oléochimie et plus particulièrement des huiles utilisées en cosmétique.

Plus particulièrement, l'invention se rapporte à un procédé d'extraction sélective des insaponifiables d'une matière lipidique d'origine végétale, cette matière lipidique renouvelable étant de l'huile de karanja.

A l'issu dudit procédé, un extrait est obtenu, ledit extrait (consistant en une solution) pouvant être incorporé dans des compositions cosmétiques ou pharmaceutiques.

Le karanja est une plante de la famille des légumineuses, originaire d'Asie. Il est particulièrement présent en Inde, au Japon, et dans le Sud de la Chine. Il s'agit d'un arbre ou d'une liane robuste mesurant de 20 à 30 m.

Le terme « karanja » au sens de la présente demande désigne notamment les espèces suivantes : *Pongamia glabra, Pongamia pinnata, Milletia pinnata, Derris indica, gadelupa pinnata, Pongamia grandifolia, Robinia mitis, Tephrosia purpurea, Tephrosia hamiltoni, Tephrosia falciformis, Tephrosia vogellii, Tephrosia lanceolata,* etc.

Différentes parties de la plante sont utilisées : les racines sont utilisées comme produit dentaire ou bien comme antiseptique local, les feuilles sont digestives et laxatives, et sont également utilisées comme antiseptique. Les écorces sont utilisées comme vermifuge et, enfin, les graines sont utilisées comme source d'huile de karanja.

Les graines de karanja comprennent environ de 27 à 39% d'huile.

L'huile de karanja est obtenue à partir des graines, par pression ou par extraction par solvant. Le tourteau, moins riche en huile que les graines, peut également être utilisé comme matière première. Les huiles obtenues possèdent généralement les mêmes caractéristiques physico-chimiques, se présentant sous la forme d'un liquide trouble, de couleur brune à orange, et d'odeur forte, désagréable.

L'huile de karanja comprend principalement des triglycérides ainsi que des acides oléiques et linoléiques, et, en plus faibles proportions : de l'acide palmitique, de l'acide stéarique, de l'acide linolénique, de l'acide arachidique ainsi que de l'acide béhénique.

L'huile de karanja, une fois extraite des graines, est largement utilisée. Dans la pharmacopée ayurvédique, elle est en particulier utilisée pour le soin de la peau, des cheveux, notamment eu égard à ses propriétés antiseptiques et antiparasitaires, elle est aussi utilisée dans le traitement de l'eczéma, du psoriasis et dans le traitement du cuir chevelu. L'huile de karanja est parfois également utilisée comme insecticide.

L'huile de karanja, outre les utilisations traditionnelles ci-dessus relatées, est également utilisée de manière plus récente comme filtre solaire, seule ou en association avec d'autres filtres solaires, dans des compositions cosmétiques. Ceci est dû notamment à la présence de certains composés insaponifiables. A titre indicatif, l'huile de karanja est composée d'environ 2 à 5% d'insaponifiables, dont les plus connus sont deux dicétones : le pongamol (CAS 482-33-3) et la karanjine (CAS 521-88-0).

De manière très générale, les pourcentages massiques moyens en pongamol et karanjine dans les huiles de karanja sont compris entre 0,3 et 0,9% pour le pongamol et entre 2 et 4% pour la karanjine.

Ces pourcentages massiques ne sont donnés qu'à titre indicatif, car ils peuvent varier largement en fonction de l'espèce, de la saison, du lieu de prélèvement, etc.

Les propriétés de protection solaire de l'huile de karanja sont essentiellement dues à la présence de pongamol, ce dernier est un filtre naturel ayant un spectre d'absorption UV similaire à celui du methoxydibenzoylmethane (CAS 70356-09-1), plus connu sous le nom de marque PARSOL 1789®.

Cependant, l'incorporation d'huile de karanja dans des compositions solaires est limitée par les caractéristiques organoleptiques de ladite huile.

Ces caractéristiques organoleptiques négatives sont essentiellement dues à la présence de karanjine.

L'élimination de tout ou d'une partie de la karanjine de l'huile de karanja a donc pour effet direct d'augmenter la quantité incorporable de cette huile dans des compositions cosmétiques solaires. Par ailleurs, le maintien d'un pourcentage massique suffisant en pongamol est important afin de préserver de bonnes propriétés solaires.

Cependant, l'élimination sélective de la karanjine est difficile, de par la parenté chimique existant entre la karanjine et le pongamol.

Dans la demande WO 2014/016349 au nom de BIOSYNTHIS, il est divulgué une composition solaire comprenant de l'huile de karanja ainsi que d'autres composés, et notamment un polyester. Dans cette demande, il n'est nullement question d'un procédé d'extraction sélective.

Dans la publication "Exhibitors Suppliers Day - USA", 1 mai 2013 (2013-05-01), pages 1-16, XP055264364 (http.//www.youbuyfrance.com/medias/press/2013-03-05-cataloguefrench-pavilion-suppliers-day 7 5 2013 59 19.pdf), il est question d'un stand BIOSYNTHIS, et notamment du produit KARANSUN®, ce produit de 2013 ne comprenant pas de diester.

La demande WO 2014114888 au nom de Jean-Noël THOREL est relative à un système de protection contre les UV et les radicaux libres, basé sur l'association d'huile de karanja et de penthaerythrityl tetra-di-t-butyl hydroxyhydrocinnamate.

La publication "FPS 40 Sun Cream for the Face", GNP D; MINTEL, 1 septembre 2008 (2008-09-01), XP002716746 est relative à une composition commerciale comprenant de l'huile de karanja ainsi que du pongamol. Du pongamol est ajouté en sus de celui compris dans l'huile de karanja, l'huile de karanja n'étant apparemment pas suffisamment concentrée en pongamol.

La demande FR 2720643 au nom de CLARINS est relative à une préparation cosmétique destinée à améliorer l'état de la peau, il n'est pas explicitement question d'un produit solaire. L'huile de karanja est exemplifiée en tant qu'ingrédient actif susceptible d'avoir un effet sur les UV.

Dans une publication commerciale (GIVAUDAN : Leading sensory innovation : Pongamia extract and karanja oil, Issue 004 April 08), les effets solaires de compositions comprenant du pongamol pur sont vantées. En page 11, le pongamol pur est caractérisé (teneur de 90% au minimum).

Dans le brevet FR 2762008 au nom de PIERRE FABRE, un procédé de désodorisation de l'huile de karanja est divulgué. D'autres applications cosmétiques de l'huile de karanja y sont également décrites, notamment des propriétés antiride et hydratante.

Dans la demande GB 2237805 au nom de UNILEVER PLC, un procédé d'extraction de dicétone est divulgué, ce procédé comporte deux étapes : extraction avec un acide organique et séparation des molécules de dicétone gênantes. Plus précisément, il s'agit d'extraction de pongamol. Il est précisé que l'huile de karanja, après retrait du pongamol, est utilisée pour la fabrication de savons, l'huile finale est donc relativement dépourvue de pongamol. S'agissant du pongamol, des cristaux se forment au cours du procédé, celui-ci ayant un point de fusion compris entre 126°C et 127,5°C, le pongamol obtenu semble donc relativement pur.

Dans la demande WO 2014195639 au nom de VALAGRO, un procédé d'extraction des insaponifiables d'une matière première lipidique renouvelable est divulgué. L'exemple 1 est tout particulièrement relatif à l'extraction sélective du pongamol et de la karanjine. Le procédé se compose principalement de deux étapes, une première étape d'extraction solide/liquide réalisée à l'aide d'un percolateur avec circulation et recirculation d'un mélange éthanol/hexane à travers des graines broyées et préalablement séchées, ainsi qu'une étape de concentration par évaporation du solvant.

En résumé, aucun document de l'art antérieur ne divulgue un procédé d'extraction sélective de la karanjine économique, respectueux de l'environnement, peu coûteux et facile à mettre en oeuvre.

Ceci est probablement dû aux difficultés que l'homme du métier rencontre pour extraire sélectivement deux molécules proches d'un point de vue structural.

Il existe donc un intérêt à disposer d'un procédé d'extraction sélective amélioré.

De manière surprenante, il a été mis en évidence qu'il était possible d'extraire sélectivement la karanjine au moyen d'un procédé présentant toutes les caractéristiques positives suivantes : économique, respectueux de l'environnement, peu coûteux et facile à mettre en oeuvre en solubilisant sélectivement le pongamol au moyen de solvants de type diesters qui sont compatibles avec les exigences de l'industrie cosmétique.

Parmi ces diesters, on considère notamment les composés de formule (I) suivante, ou leurs mélanges :

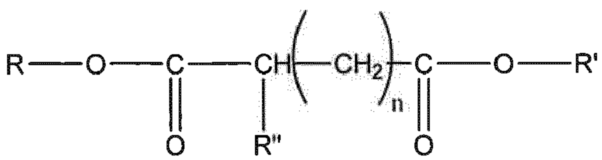

Formule (I)

dans laquelle :

- n est compris entre 0 et 19 ;
- R et R' identiques ou différents sont des alkyls provenant d'une estérification par un alcool linéaire ou ramifié de formule brute $C_xH_{2x+2}O$, x étant compris entre 1 et 30, préférentiellement entre 1 et 20, préférentiellement entre 1 et 10.
- R" est soit un atome d'hydrogène soit un groupement alkyle en $C_1$-$C_3$.

Ces diesters ont des points éclairs supérieurs à 70°C et ne présentent donc pas les dangers des COV dont les points éclairs sont inférieurs à 70°C.

Dans le cadre de la présente invention, certains diesters sont particulièrement préférés, comme par exemple les sébacates (n=7), les adipates (n=3), les succinates (n=1), les dodécanedioates (n=9), les azélates (n=6), les glutarates (n=2), les malonates (n=0), etc.

L'invention concerne un ingrédient cosmétique, caractérisé en ce qu'il consiste en une solution d'au moins un extrait d'huile de karanja comprenant du pongamol (CAS 484-33-3) et de la karanjine (CAS 521-88-0) dans au moins

un solvant choisi dans le groupe des diesters de formule (I) suivante, ou leurs mélanges :

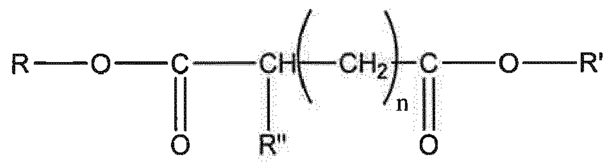

Formule (I)

dans laquelle :

- n est compris entre 0 et 19 ;
- R et R' identiques ou différents sont choisis dans le groupe des alkyls issus d'un alcool linéaire ou ramifié de formule brute $C_xH2_{x+2}O$ avant l'estérification, x étant compris entre 1 et 30, préférentiellement entre 1 et 20, préférentiellement entre 1 et 10.
- R" est soit un atome d'hydrogène soit un groupement alkyle en $C_1$-$C_3$.

L'invention concerne également un procédé de précipitation sélective du pongamol (CAS 484-33-3) dans une huile de karanja comprenant du pongamol (CAS 484-33-3) et de la karanjine (CAS 521-88-0), caractérisé en ce qu'il comprend:

1) au moins une étape d'addition à ladite au moins une huile de karanja d'au moins un solvant choisi dans le groupe des diesters de formule (I) suivante, ou leurs mélanges :

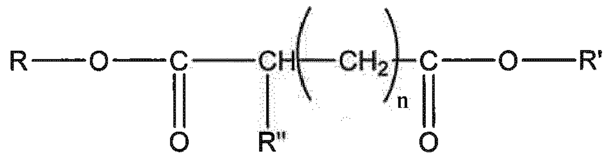

Formule (I)

dans laquelle :

- n est compris entre 0 et 19 ;
- R et R' identiques ou différents sont des alkyls provenant d'une estérification par un alcool linéaire ou ramifié de formule brute $C_xH2_{x+2}O$, x étant compris entre 1 et 30, préférentiellement entre 1 et 20, préférentiellement entre 1 et 10.
- R" est soit un atome d'hydrogène soit un groupement alkyle en $C_1$-$C_3$.

ladite addition ayant pour effet de former une phase légère (surnageant) sous forme de solution et une phase lourde sous forme de précipité ; et,

2) au moins une étape de séparation des deux phases obtenues.

L'invention concerne également une formulation cosmétique, caractérisée en ce qu'elle comprend :

- un ingrédient cosmétique selon l'invention ;
- un véhicule cosmétiquement acceptable.

L'invention concerne également l'utilisation d'une formulation cosmétique selon l'invention telle que décrite ci-dessus, en tant que formulation cosmétique solaire.

L'invention concerne également l'utilisation d'une formulation cosmétique selon l'invention telle que décrite ci-dessus, en tant que formulation cosmétique anti-âge.

Outre les intérêts écologiques et économiques, le procédé selon l'invention permet l'obtention avec un procédé comprenant peu d'étapes, d'une solution enrichie en pongamol prête à être directement formulée sans nécessiter une étape de fusion et dissolution à chaud du pongamol avant incorporation dans une formulation cosmétique comme cela doit être fait lorsqu'on utilise du pongamol pur.

Du point de vue économique, du respect de l'environnement et du coût, le procédé qui peut être réalisé à température ambiante permet de réaliser une économie de ressources, dans la mesure où aucun solvant n'est éliminé durant le procédé. En outre, aucun solvant toxique n'est utilisé, en particulier, aucun solvant inflammable classé comme COV léger (points éclair < 70°C) n'est utilisé.

Le procédé permet également d'utiliser des solvants biosourcés tels que les sébacates, les succinates, et les azélates présentant un meilleur impact environnemental que leurs homologues d'origine fossile. En outre, l'extraction de la fraction insaponifiable préserve les triglycérides, lesquels peuvent être à leur tour valorisés, car le procédé ne comprend aucune étape de saponification ou d'hydrolyse.

Du point de vue de la facilité de mise en oeuvre, le procédé comprend un nombre limité d'étapes relativement simples à mettre en oeuvre. En particulier, il ne présente aucune étape de conditionnement de la graine (séchage, broyage, aplatissage, etc.) nécessitée par exemple par le procédé décrit dans la demande WO 2014195639 au nom de VALAGRO.

Un certain nombre de définitions sont données ci-après.

On appelle « huile de karanja » toute huile issue d'une plante de la famille des karanjas. Il peut notamment s'agir des espèces de plante suivantes : *Pongamia glabra, Pongamia pinnata, Milletia pinnata, Derris indica, gadelupa pinnata, Pongamia grandifolia, Robinia mitis, Tephrosia purpurea, Tephrosia hamiltoni, Tephrosia falciformis, Tephrosia vogellii, Tephrosia lanceolata,* etc. L'huile de karanja peut être « brute », c'est-à-dire obtenue directement à partir des graines par pression à froid ou extraction par solvant avec élimination du solvant.

L'huile de karanja peut également subir des traitements pour la purifier en éliminant notamment des impuretés, la désodoriser ou la décolorer.

Il peut par exemple s'agir d'une ou plusieurs distillation(s) moléculaire(s) et/ou d'une démucilagination et/ou d'une ou plusieurs désodorisation(s).

On appelle « phase légère (surnageant) » la phase légère issue de l'étape de précipitation sélective. Il s'agit d'une solution. Elle est séparée de la phase lourde (précipité) par des méthodes bien connues de l'homme du métier, pouvant par exemple être une centrifugation ou une filtration. La phase légère (surnageant) présente un intérêt tout particulier, dans la mesure où une fois séparée de la phase lourde (précipité), la phase légère (surnageant) qui est une solution constitue l'ingrédient cosmétique selon l'invention.

On appelle « phase lourde (précipité) » la phase lourde issue de l'étape de précipitation sélective. Elle est séparée de la phase légère (surnageant) par des méthodes bien connues de l'homme du métier, pouvant par exemple être une centrifugation ou une filtration.

On appelle « précipitation sélective » une précipitation permettant la précipitation en particulier d'une espèce chimique à partir d'un mélange. Dans le procédé de précipitation sélective selon l'invention, la karanjine est préférentiellement précipitée par rapport au pongamol. Par exemple, lorsqu'au cours d'une étape de précipitation, le précipitât est composé de 61,5% de karanjine et de seulement 4,3% de pongamol, il s'agit d'une précipitation sélective de la karanjine.

On appelle « pourcentage massique » (exprimé en %) le rapport entre la masse d'un constituant d'un mélange et la masse totale du mélange. Par exemple, si un constituant i d'un mélange a une masse de 1 kg et que le mélange a une masse de 100 kg, le pourcentage massique du constituant i est 1%. Par exemple, lorsqu'un ingrédient cosmétique selon l'invention est caractérisé en ce que le pourcentage massique en pongamol dans la solution (dont l'ingrédient cosmétique est entièrement constitué) est de 3 %, cela signifie que dans 100 g d'ingrédient cosmétique/de solution, il y a 3 g de pongamol.

On appelle « rendement d'extraction », le rapport entre la masse d'un composant avant une étape (par exemple, une étape de précipitation sélective) et la masse du même composant après ladite étape. Il peut notamment être exprimé en pourcentage, et est compris entre 0% et 100%. Par exemple, le rendement d'extraction du pongamol lors de la précipitation est calculé comme suit : masse de pongamol dans l'huile de karanja avant la précipitation/masse de pongamol dans la solution obtenue à l'issue de la précipitation. Lorsqu'on parle de « rendement d'extraction dans une phase », il s'agit du rapport entre la masse d'un composant dans la phase et la masse du même composant avant l'étape ayant abouti à la formation de la phase.

On appelle « multiplication du rapport $m_{pongamol}/m_{karanjine}$ » la multiplication du rapport des masses $m_{pongamol}/m_{karanjine}$ lors d'une ou de plusieurs étapes du procédé, exprimée comme suit :

$$Multiplication\ du\ rapport\ \frac{mpongamol}{mkaranjine} = \frac{Rapport\ \frac{mpongamol}{mkaranjine}\ \mathbf{après}\ la\ ou\ les\ étape(s)}{Rapport\ \frac{mpongamol}{mkaranjine}\ \mathbf{avant}\ la\ ou\ les\ étape(s)}$$

Par exemple, il peut s'agir de la multiplication du rapport massique $m_{pongamol}/m_{karanjine}$ lors des étapes 1) et

2), la comparaison est alors faite entre le rapport massique $m_{pongamol}/m_{karanjine}$ avant l'étape 1) et le rapport $m_{pongamol}/m_{karanjine}$ après l'étape 2) ; si avant l'étape 1), le rapport $m_{pongamol}/m_{karanjine}$ est de 0,71, et après l'étape 2), le rapport $m_{pongamol}/m_{karanjine}$ (dans la solution issue de la récupération de la phase légère ou surnageant) est de 1,75, alors la multiplication du rapport $m_{pongamol}/m_{karanjine}$ se calcule comme suit : 1,75/0,71 = 2,46. Le rapport $m_{pongamol}/m_{karanjine}$ a été multiplié par un facteur 2,46 lors des étapes 1) et 2).

On appelle « photostabilité » la stabilité d'un composé quelconque pouvant être notamment un ingrédient cosmétique selon l'invention vis-à-vis de la dégradation induite par un rayonnement lumineux ou photodégradation.

L'invention concerne un ingrédient cosmétique, caractérisé en ce qu'il consiste en une solution d'au moins un extrait d'huile de karanja comprenant du pongamol (CAS 484-33-3) et de la karanjine (CAS 521-88-0) dans au moins un solvant choisi dans le groupe des diesters de formule (I) suivante, ou leurs mélanges :

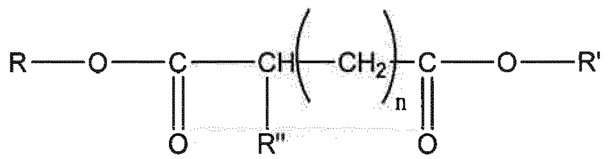

Formule (I)

dans laquelle :

- n est compris entre 0 et 19 ;
- R et R' identiques ou différents sont des alkyls provenant d'une estérification par un alcool linéaire ou ramifié de formule brute $C_xH2_{x+2}O$, x étant compris entre 1 et 30, préférentiellement entre 1 et 20, préférentiellement entre 1 et 10.
- R" est soit un atome d'hydrogène soit un groupement alkyle en $C_1$-$C_3$.

Dans un mode de réalisation, l'ingrédient cosmétique selon l'invention est caractérisé en ce que R" est un atome d'hydrogène.

Dans un mode de réalisation, l'ingrédient cosmétique selon l'invention est caractérisé en ce que R" est un méthyle.

Dans un mode de réalisation, l'ingrédient cosmétique selon l'invention est caractérisé en ce que x est compris entre 1 et 20.

Dans un mode de réalisation, l'ingrédient cosmétique selon l'invention est caractérisé en ce que x est compris entre 1 et 10.

Dans un mode de réalisation, l'ingrédient cosmétique selon l'invention est caractérisé en ce que n est compris entre 1 et 10.

Dans un mode de réalisation, l'ingrédient cosmétique selon l'invention est caractérisé en ce que R est identique à R'.

Dans un mode de réalisation, l'ingrédient cosmétique selon l'invention est caractérisé en ce que R est différent de R'.

Dans un mode de réalisation, l'ingrédient cosmétique selon l'invention est caractérisé en ce que ledit au moins un solvant est choisi dans le groupe constitué des sébacates (n=7), des adipates (n=3), des succinates (n=1), des dodécanedioates (n=9), des azélates (n=6), des glutarates (n=2), des malonates (n=0), et de leurs mélanges.

Dans un mode de réalisation, l'ingrédient cosmétique selon l'invention est caractérisé en ce que ledit au moins un solvant est choisi dans le groupe constitué des sébacates (n=7).

Dans un mode de réalisation, l'ingrédient cosmétique selon l'invention est caractérisé en ce que ledit au moins un solvant est choisi dans le groupe constitué des adipates (n=3).

Dans un mode de réalisation, l'ingrédient cosmétique selon l'invention est caractérisé en ce que ledit au moins un solvant est choisi dans le groupe constitué des des succinates (n=1).

Dans un mode de réalisation, l'ingrédient cosmétique selon l'invention est caractérisé en ce que ledit au moins un solvant est choisi dans le groupe constitué des des dodécanedioates (n=9).

Dans un mode de réalisation, l'ingrédient cosmétique selon l'invention est caractérisé en ce que ledit au moins un solvant est choisi dans le groupe constitué des des azélates (n=6).

Dans un mode de réalisation, l'ingrédient cosmétique selon l'invention est caractérisé en ce que ledit au moins un solvant est choisi dans le groupe constitué des des glutarates (n=2).

Dans un mode de réalisation, l'ingrédient cosmétique selon l'invention est caractérisé en ce que ledit au moins un solvant est choisi dans le groupe constitué des des malonates (n=0).

Les sébacates sont des solvants largement utilisés. Ils sont des dérivés de l'acide sébacique, ou acide décanedioïque (CAS 111-20-6) qui est un acide dicarboxylique à 10 carbones de formule suivante :

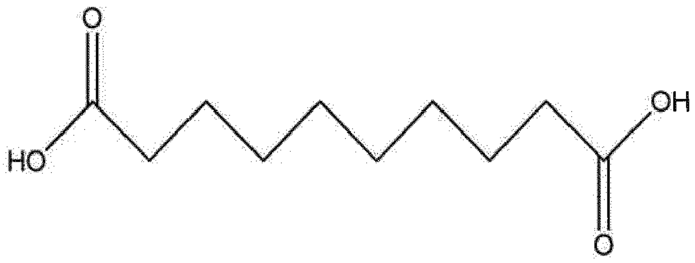

L'acide sébacique peut être doublement estérifié par l'emploi de deux alcools identiques ou différents.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins un solvant est choisi dans le groupe constitué du sébacate de dioctyle (CAS 122-62-3), du sébacate de diéthyle (CAS 110-40-7), du sébacate de dibutyle (CAS 109-43-3), du sébacate de diisopropyle (CAS 7491-02-3) et leurs mélanges.

Dans un mode de réalisation, l'ingrédient cosmétique selon l'invention est caractérisé en ce que ledit au moins un solvant est choisi dans le groupe constitué du sébacate de diéthyle (CAS 110-40-7), du sébacate de dibutyle (CAS 109-43-3), du sébacate de diisopropyle (CAS 7491-02-3) et leurs mélanges.

Dans un mode de réalisation, l'ingrédient cosmétique selon l'invention est caractérisé en ce que ledit au moins un solvant est le sébacate de diéthyle (CAS 110-40-7).

Les diesters d'acide sébacique sont utilisés en cosmétologie. Dans la demande WO 9850005 au nom de MEDLOGIC GLOBAL CORPORATION, le sébacate de diéthyle est cité en tant que solvant, et le sébacate de diisopropyle et le sébacate de dibutyle sont cités en tant qu'émollients.

Dans le brevet US 5152983 au nom de CHESEBROUGH-POND'S USA CO, le sébacate de dibutyle est cité en tant qu'émollient, et il est prévu l'adjonction de pongamol dans la composition. Dans la composition de l'exemple 1, une composition solaire comprenant du pongamol est testée.

Les adipates sont des solvants largement utilisés. Ils sont des dérivés de l'acide adipique (CAS 124-04-9) qui est un acide dicarboxylique aliphatique de formule suivante :

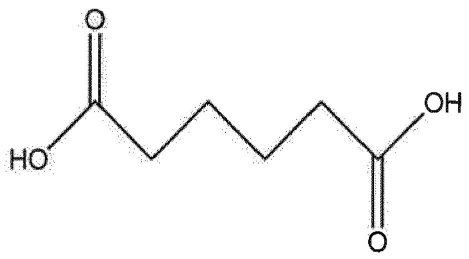

L'acide adipique peut être doublement estérifié par l'emploi de deux alcools identiques ou différents.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, l'ingrédient cosmétique selon l'invention est caractérisé en ce que ledit au moins un solvant est un acide adipique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide adipique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins un solvant est choisi dans le groupe constitué de l'adipate de dihexyle (CAS 2091-24-9), de l'adipate de diisostearyle (CAS 62479-36-1), de l'adipate de dicapryle (CAS 108-63-4), de l'adipate de di-C12-15 alkyle, de l'adipate de ditridecyle (CAS 16958-92-2), de l'adipate de dicetyle (CAS 26720-21-8), de l'adipate de diisopropyle (CAS 6938-94-9), de l'adipate de diisobutyle (CAS 141-04-8), de l'adipate de diéthylhexyle (CAS 103-23-1), de l'adipate de diisooctyle (CAS 1330-86-5), de l'adipate de diisononyle (CAS 33703-08-1), de l'adipate de diisodecyle (CAS 27178-16-1), de l'adipate de diéthyle (CAS 141-28-6), de l'adipate de diméthyle (CAS 627-93-0), de l'adipate de dihexyldecyle (CAS 57533-90-1), de l'adipate de diheptylundecyle (CAS 155613-91-5), de l'adipate de dipropyle (CAS 106-19-4), de l'adipate de dioctyldodecyle (CAS 85117-94-8), de l'adipate de dibutyle (CAS 105-99-7), de l'adipate de diisocetyle (CAS 57533-90-1), de l'adipate de dioctyle (CAS 123-79-5) et leurs mélanges.

Dans un mode de réalisation, l'ingrédient cosmétique selon l'invention est caractérisé en ce que ledit au moins un solvant est choisi dans le groupe constitué de l'adipate de diisopropyle (CAS 6938-94-9), de l'adipate de dibutyle (CAS 105-99-7), de l'adipate de dioctyle (CAS 123-79-5) et leurs mélanges.

Les succinates sont des solvants largement utilisés. Ils sont des dérivés de l'acide succinique (CAS 110-15-6) de formule suivante :

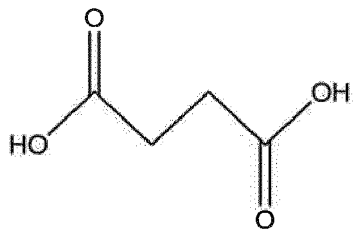

L'acide succinique peut être doublement estérifié par l'emploi de deux alcools identiques ou différents.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, l'ingrédient cosmétique selon l'invention est caractérisé en ce que ledit au moins un solvant est un acide succinique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide succinique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins un solvant est choisi dans le groupe constitué du succinate de didécyle (CAS 10595-82-1), du succinate de diméthyle (CAS 106-65-0), du succinate de diéthyle (CAS 123-25-1), du succinate de dicapryle (CAS 14491-66-8), du succinate de dicétéaryle (CAS 93280-98-9), du succinate de diisobutyle (CAS 925-06-4), du succinate de diéthylhexyle (CAS 2915-57-3) et leurs mélanges.

Dans un mode de réalisation, l'ingrédient cosmétique selon l'invention est caractérisé en ce que ledit au moins un solvant est le succinate de diéthylhexyle (CAS 2915-57-3).

Les 2-méthyl succinates sont des dérivés de l'acide 2-méthyl succinique (CAS 498-21-5) de formule suivante :

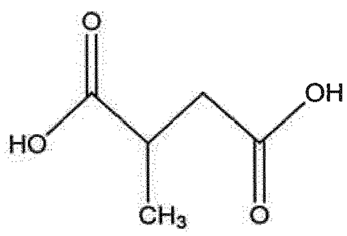

L'acide 2-méthyl succinique peut être doublement estérifié par l'emploi de deux alcools identiques ou différents.

Dans un mode de réalisation, l'ingrédient cosmétique selon l'invention est caractérisé en ce que ledit au moins un solvant est un acide 2-méthyl succinique doublement estérifié par deux alcools identiques ou différents, chacun des deux alcools étant un alcool linéaire ou ramifié, ou un mélange d'isomères dans n'importe quelles proportions le cas

échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, l'ingrédient cosmétique selon l'invention est caractérisé en ce que ledit au moins un solvant est un acide 2-méthyl succinique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide 2-méthyl succinique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins un solvant est choisi dans le groupe constitué du 2-méthyl succinate de diéthyle (CAS 4676-51-1), du 2-méthyl succinate de 1-éthyle et de 4-méthyle (CAS 204125-41-7), du 2-méthyl succinate de 1-methyle et de 4-méthyle (CAS 606491-29-6), du 2-méthyl succinate de dipropyle (CAS 56108-32-8), du 2-méthyl succinate de diisopropyle (CAS 75906-62-6), du 2-méthyl succinate de 1-butyle et de 4-méthyle (CAS 878209-18-8), du 2-méthyl succinate de di(2-methylpropyle) (CAS 18447-89-7), du 2-méthyl succinate de 1-pentyle et de 4-méthyle (CAS 204125-40-6), du 2-méthyl succinate de 1-méthyl et de 4-hexyl (CAS 214280-22-5), du 2-méthyl succinate de di(1-méthylpropyle) (CAS 57983-31-0), du 2-méthylsuccinate de di(1,1-diméthyléthyle) (CAS 108763-17-3), du 2-méthyl succinate de dipentyle (CAS 56108-33-9), du 2-méthyl succinate de dihexyle (CAS 32774-96-2), du 2-méthyl succinate de diheptyle (CAS 51191-78-7), du 2-méthyl succinate de 1-méthyle et de 4-dodécyle (CAS 214280-27-0), du 2-methyl succinate de dioctyle (CAS 131787-12-7), du 2-méthyl succinate de 1-méthyle et de 4-octadécyle, du 2-méthyl succinate de didécyle, du 2-méthyl succinate de didodécyle ou lauryle, du 2-méthylsuccinate de décanyle, du 2-méthylsuccinate de 2-éthylhexanyle , de leurs isomères et mélanges d'isomères, et de leurs mélanges.

Les dodécanedioates sont des solvants largement utilisés. Ils sont des dérivés de l'acide dodécanedioïque (CAS 693-23-2) qui est un acide dicarboxylique aliphatique de formule suivante :

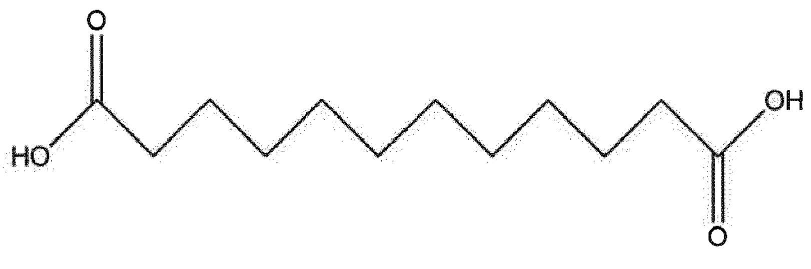

L'acide dodécanedioïque peut être doublement estérifié par l'emploi de deux alcools identiques ou différents.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, l'ingrédient cosmétique selon l'invention est caractérisé en ce que ledit au moins un solvant est un acide dodécanedioïque doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les

heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide dodécanedioïque doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins un solvant est choisi dans le groupe constitué du dodecanedioate de dioctyldodecyle (CAS 129423-55-8), du dodecanedioate de diisocetyle (CAS 131252-83-0) et leurs mélanges.

Les azélates sont des solvants largement utilisés. Ils sont des dérivés de l'acide azélaïque (CAS 123-99-9) de formule suivante :

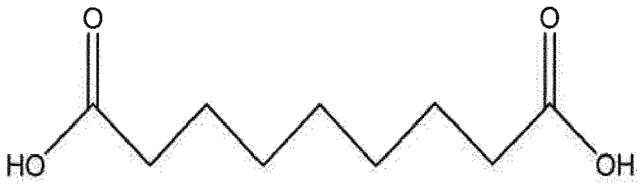

L'acide azélaïque peut être doublement estérifié par l'emploi de deux alcools identiques ou différents.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, l'ingrédient cosmétique selon l'invention est caractérisé en ce que ledit au moins un solvant est un acide azélaïque doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide azélaïque doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins

un solvant est choisi dans le groupe constitué de l'azélate de diméthyle (CAS 1732-10-1), de l'azélate de di(2-éthylhexyle) et leurs mélanges.

Les glutarates sont des solvants largement utilisés. Ils sont des dérivés de l'acide glutarique (CAS 110-94-1) de formule suivante :

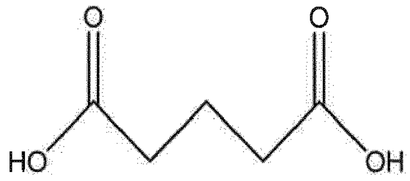

L'acide glutarique peut être doublement estérifié par l'emploi de deux alcools identiques ou différents.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, l'ingrédient cosmétique selon l'invention est caractérisé en ce que ledit au moins un solvant est un acide glutarique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide glutarique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol

(CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins un solvant est choisi dans le groupe constitué du glutarate de diméthyle (CAS 1119-40-0), du glutarate de diisobutyle (CAS 71195-64-7), du glutarate de diisostéaryle, du 2-méthylglutarate de diméthyle (CAS 14035-94-0) et leurs mélanges.

Les malonates sont des solvants largement utilisés. Ils sont des dérivés de l'acide malonique (CAS 141-82-2) de formule suivante :

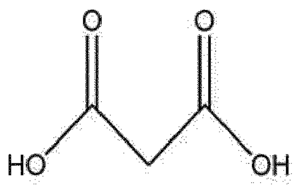

L'acide malonique peut être doublement estérifié par l'emploi de deux alcools identiques ou différents.

Dans un mode de réalisation, l'ingrédient cosmétique selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement

en $C_1$-$C_{10}$.

Dans un mode de réalisation, l'ingrédient cosmétique selon l'invention est caractérisé en ce que ledit au moins un solvant est un acide malonique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, l'ingrédient cosmétique selon l'invention est caractérisé en ce que ledit au moins un solvant est un acide malonique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, l'ingrédient cosmétique selon l'invention est caractérisé en ce que ledit au moins un solvant est le malonate de diéthyle (CAS 105-53-3).

Dans un mode de réalisation, l'ingrédient cosmétique selon l'invention est caractérisé en ce que ledit extrait d'huile de karanja est extrait d'une espèce choisie dans le groupe constitué de *Pongamia glabra, Pongamia pinnata, Milletia pinnata, Derris indica, gadelupa pinnata, Pongamia grandifolia, Robinia mitis, Tephrosia purpurea, Tephrosia hamiltoni, Tephrosia falciformis, Tephrosia vogellii* et *Tephrosia lanceolata.*

Dans un mode de réalisation, l'ingrédient cosmétique selon l'invention est caractérisé en ce que ledit extrait d'huile de karanja est extrait d'une espèce choisie dans le groupe constitué de *Pongamia glabra* et *Milletia pinnata.*

Dans un mode de réalisation, l'ingrédient cosmétique selon l'invention est caractérisé en ce que ledit extrait d'huile de karanja est extrait de l'espèce *Milletia pinnata.*

Dans un mode de réalisation, l'ingrédient cosmétique selon l'invention est caractérisé en ce que le pourcentage massique en ledit au moins un solvant dans ladite solution est compris entre 20% et 60%.

Dans un mode de réalisation, l'ingrédient cosmétique selon l'invention est caractérisé en ce que le pourcentage massique en ledit au moins un solvant dans ladite solution est compris entre 40% et 60%.

Dans un mode de réalisation, l'ingrédient cosmétique selon l'invention est caractérisé en ce que le pourcentage massique en ledit au moins un solvant dans ladite solution est d'environ 50%.

Dans un mode de réalisation, l'ingrédient cosmétique selon l'invention est caractérisé en ce que le pourcentage massique en pongamol dans ladite solution est supérieur à 1,30%.

Dans un mode de réalisation, l'ingrédient cosmétique selon l'invention est caractérisé en ce que le pourcentage massique en pongamol dans ladite solution est supérieur à 1,20%.

Dans un mode de réalisation, l'ingrédient cosmétique selon l'invention est caractérisé en ce que le pourcentage massique en karanjine dans ladite solution est inférieur à 6%.

Dans un mode de réalisation, l'ingrédient cosmétique selon l'invention est caractérisé en ce que le pourcentage massique en karanjine dans ladite solution est inférieur à 5%.

Dans un mode de réalisation, l'ingrédient cosmétique selon l'invention est caractérisé en ce que le pourcentage massique en karanjine dans ladite solution est inférieur à 4,5%.

Dans un mode de réalisation, l'ingrédient cosmétique selon l'invention est caractérisé en ce que le pourcentage massique en karanjine dans ladite solution est inférieur à 4,3%.

Dans un mode de réalisation, l'ingrédient cosmétique selon l'invention est caractérisé en ce que le rapport $m_{pongamol}/m_{karanjine}$ dans ladite solution est supérieur à 0,5.

Dans un mode de réalisation, l'ingrédient cosmétique selon l'invention est caractérisé en ce que le rapport $m_{pongamol}/m_{karanjine}$ dans ladite solution est supérieur à 0,55.

L'invention concerne également un procédé de précipitation sélective du pongamol (CAS 484-33-3) dans une huile de karanja comprenant du pongamol (CAS 484-33-3) et de la karanjine (CAS 521-88-0), caractérisé en ce qu'il comprend :

1) au moins une étape d'addition à ladite au moins une huile de karanja d'au moins un solvant choisi dans le groupe des diesters de formule (I) suivante, ou leurs mélanges :

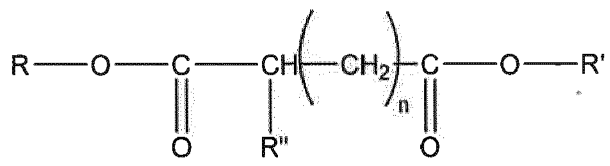

Formule (I)

dans laquelle :

- n est compris entre 0 et 19 ;
- R et R' identiques ou différents sont des alkyls provenant d'une estérification par un alcool linéaire ou ramifié de formule brute $C_xH_{2x+2}O$, x étant compris entre 1 et 30, préférentiellement entre 1 et 20, préférentiellement entre 1 et 10.
- R" est soit un atome d'hydrogène soit un groupement alkyle en $C_1$-$C_3$.

ladite addition ayant pour effet de former une phase légère sous forme d'une solution et une phase lourde sous forme de précipité ; et
2) au moins une étape de séparation des deux phases obtenues.

Dans un mode de réalisation, le procédé selon l'invention est caractérisé en ce que le rendement d'extraction de ladite karanjine dans ladite phase légère sous forme de solution est inférieur à 50%.

Dans un mode de réalisation, le procédé selon l'invention est caractérisé en ce que le rendement d'extraction dudit pongamol dans ladite phase légère sous forme de solution est supérieur à 60%.

Dans un mode de réalisation, le procédé selon l'invention est caractérisé en ce qu'il entraîne une multiplication du rapport massique $m_{pongamol}/m_{karanjine}$ d'un facteur supérieur à 2.

Dans un mode de réalisation, le procédé selon l'invention est caractérisé en ce que lors de ladite étape 1), la proportion massique dudit au moins un solvant introduit dans ladite au moins une huile de karanja est comprise entre 60/40 et 20/80.

Dans un mode de réalisation, le procédé selon l'invention est caractérisé en ce que les étapes 1) et 2) sont réalisées à température ambiante, par exemple à environ 25°C.

Dans un mode de réalisation, le procédé selon l'invention est caractérisé en ce que ladite huile de karanja est issue d'une espèce choisie dans le groupe constitué de *Pongamia glabra, Pongamia pinnata, Milletia pinnata, Derris indica, gadelupa pinnata, Pongamia grandifolia, Robinia mitis, Tephrosia purpurea, Tephrosia hamiltoni, Tephrosia falciformis, Tephrosia vogellii* et *Tephrosia lanceolata.*

Dans un mode de réalisation, le procédé selon l'invention est caractérisé en ce que ladite huile de karanja est issue d'une espèce choisie dans le groupe constitué de *Pongamia glabra* et *Milletia pinnata.*

Dans un mode de réalisation, le procédé selon l'invention est caractérisé en ce que ladite huile de karanja est issue de l'espèce *Millettia pinnata.*

Dans un mode de réalisation, le procédé selon l'invention est caractérisé en ce que ladite huile de karanja est une huile brute.

Dans un mode de réalisation, le procédé selon l'invention est caractérisé en ce qu'il comprend en outre au moins une étape préalable de désodorisation de ladite au moins une huile de karanja et/ou au moins une étape préalable de distillation de ladite au moins une huile de karanja, lesdites étapes, lorsqu'elles sont toutes deux présentes, pouvant être effectuées dans n'importe quel ordre.

Dans un mode de réalisation, le procédé selon l'invention est caractérisé en ce qu'il comprend en outre:

3) au moins une deuxième étape d'addition à ladite solution issue de ladite étape 2) d'au moins un solvant choisi dans le groupe des diesters de formule (I) suivante, ou leurs mélanges :

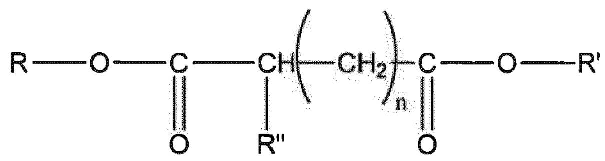

Formule (I)

dans laquelle :

- n est compris entre 0 et 19 ;
- R et R' identiques ou différents sont des alkyls provenant d'une estérification par un alcool linéaire ou ramifié de formule brute $C_xH_{2x+2}O$, x étant compris entre 1 et 30, préférentiellement entre 1 et 20, préférentiellement entre 1 et 10.
- R" est soit un atome d'hydrogène soit un groupement alkyle en $C_1$-$C_3$.

ladite deuxième étape d'addition ayant pour effet de former une phase légère sous forme de solution et une phase lourde sous forme de précipité ; et,

4) une deuxième étape de séparation des deux phases obtenues ;

et en ce que le au moins un solvant de l'étape 3) est identique ou différent du au moins un solvant de l'étape 1).

Dans un mode de réalisation, le procédé selon l'invention est caractérisé en ce que ledit au moins un solvant est choisi dans le groupe constitué des sébacates (n=7), des adipates (n=3), des succinates (n=1), des dodécanedioates (n=9), des azélates (n=6), des glutarates (n=2), des malonates (n=0), et de leurs mélanges.

Dans un mode de réalisation, le procédé selon l'invention est caractérisé en ce que ledit au moins un solvant est choisi dans le groupe constitué des sébacates (n=7).

Dans un mode de réalisation, le procédé selon l'invention est caractérisé en ce que ledit au moins un solvant est le sébacate de diéthyle (CAS 110-40-7).

Dans un mode de réalisation, le procédé selon l'invention est caractérisé en ce que R" est un atome d'hydrogène.

Dans un mode de réalisation, le procédé selon l'invention est caractérisé en ce que R" est un méthyle.

Dans un mode de réalisation, le procédé selon l'invention est caractérisé en ce que x est compris entre 1 et 20.

Dans un mode de réalisation, le procédé selon l'invention est caractérisé en ce que x est compris entre 1 et 10.

Dans un mode de réalisation, le procédé selon l'invention est caractérisé en ce que n est compris entre 1 et 10.

Dans un mode de réalisation, le procédé selon l'invention est caractérisé en ce que R est identique à R'.

Dans un mode de réalisation, le procédé selon l'invention est caractérisé en ce que R est différent de R'.

Dans un mode de réalisation, le procédé selon l'invention est caractérisé en ce que ledit au moins un solvant est choisi dans le groupe constitué des sébacates (n=7), des adipates (n=3), des succinates (n=1), des dodécanedioates (n=9), des azélates (n=6), des glutarates (n=2), des malonates (n=0), et de leurs mélanges.

Dans un mode de réalisation, le procédé selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide sébacique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide sébacique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS

584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est choisi dans le groupe constitué du sébacate de dioctyle (CAS 122-62-3), du sébacate de diéthyle (CAS 110-40-7), du sébacate de dibutyle (CAS 109-43-3), du sébacate de diisopropyle (CAS 7491-02-3) et leurs mélanges.

Dans un mode de réalisation, le procédé selon l'invention est caractérisé en ce que ledit au moins un solvant est choisi dans le groupe constitué du sébacate de diéthyle (CAS 110-40-7), du sébacate de dibutyle (CAS 109-43-3), du sébacate de diisopropyle (CAS 7491-02-3) et leurs mélanges.

Dans un mode de réalisation, le procédé selon l'invention est caractérisé en ce que ledit au moins un solvant est le sébacate de diéthyle (CAS 110-40-7).

Dans un mode de réalisation, le procédé selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide adipique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide adipique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est choisi dans le groupe constitué de l'adipate de dihexyle (CAS 2091-24-9), de l'adipate de diisostearyle (CAS 62479-36-1), de l'adipate de dicapryle (CAS 108-63-4), de l'adipate de di-C12-15 alkyle, de l'adipate de ditridecyle (CAS 16958-92-2), de l'adipate de dicetyle (CAS 26720-21-8), de l'adipate de diisopropyle (CAS 6938-94-9), de l'adipate de diisobutyle (CAS 141-04-8), de l'adipate de diéthylhexyle (CAS 103-23-1), de l'adipate de diisooctyle (CAS 1330-86-5), de l'adipate de diisononyle (CAS 33703-08-1), de l'adipate de diisodecyle (CAS 27178-16-1), de l'adipate de diéthyle (CAS 141-28-6), de l'adipate de diméthyle (CAS 627-93-0), de l'adipate de dihexyldecyle (CAS 57533-90-1), de l'adipate de diheptylundecyle (CAS 155613-91-5), de l'adipate de dipropyle (CAS 106-19-4), de l'adipate de dioctyldodecyle (CAS 85117-94-8), de l'adipate de dibutyle (CAS 105-99-7), de l'adipate de diisocetyle (CAS 57533-90-1), de l'adipate de dioctyle (CAS 123-79-5) et leurs mélanges.

Dans un mode de réalisation, le procédé selon l'invention est caractérisé en ce que ledit au moins un solvant est choisi dans le groupe constitué de l'adipate de diisopropyle (CAS 6938-94-9), de l'adipate de dibutyle (CAS 105-99-7), de l'adipate de dioctyle (CAS 123-79-5) et leurs mélanges.

Dans un mode de réalisation, le procédé selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide succinique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule

brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide succinique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est choisi dans le groupe constitué du succinate de didécyle (CAS 10595-82-1), du succinate de diméthyle (CAS 106-65-0), du succinate de diéthyle (CAS 123-25-1), du succinate de dicapryle (CAS 14491-66-8), du succinate de dicétéaryle (CAS 93280-98-9), du succinate de diisobutyle (CAS 925-06-4), du succinate de diéthylhexyle (CAS 2915-57-3) et leurs mélanges.

Dans un mode de réalisation, le procédé selon l'invention est caractérisé en ce que ledit au moins un solvant est le succinate de diéthylhexyle (CAS 2915-57-3).

Dans un mode de réalisation, le procédé selon l'invention est caractérisé en ce que ledit au moins un solvant est un acide 2-méthyl succinique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide 2-méthyl succinique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide 2-méthyl succinique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est choisi dans le groupe constitué du 2-méthyl succinate de diéthyle (CAS 4676-51-1), du 2-méthyl succinate de 1-éthyle et de 4-méthyle (CAS 204125-41-7), du 2-méthyl succinate de 1-methyle et de 4-méthyle (CAS 606491-29-6), du 2-méthyl succinate de dipropyle (CAS 56108-32-8), du 2-méthyl succinate de diisopropyle (CAS 75906-62-6), du 2-méthyl succinate de 1-butyle et de 4-méthyle (CAS 878209-18-8), du 2-méthyl succinate de di(2-methylpropyle) (CAS 18447-89-7), du 2-méthyl succinate de 1-pentyle et de 4-méthyle (CAS 204125-40-6), du 2-méthyl succinate de 1-méthyl et de 4-hexyl (CAS 214280-22-5), du 2-méthyl succinate de di(1-méthylpropyle) (CAS 57983-31-0), du 2-méthylsuccinate de di(1,1-diméthyléthyle) (CAS 108763-17-3), du 2-méthyl succinate de dipentyle (CAS 56108-33-9), du 2-méthyl succinate de dihexyle (CAS 32774-96-2), du 2-méthyl succinate de diheptyle (CAS 51191-78-7), du 2-méthyl succinate de 1-méthyle et de 4-dodécyle (CAS 214280-27-0), du 2-methyl succinate de dioctyle (CAS 131787-12-7), du 2-méthyl succinate de 1-méthyle et de 4-octadécyle, du 2-méthyl succinate de didécyle, du 2-méthyl succinate de didodécyle ou lauryle, du 2-méthylsuccinate de décanyle, du 2-méthylsuccinate de 2-éthylhexanyle, de leurs isomères et mélanges

d'isomères, et de leurs mélanges.

Dans un mode de réalisation, le procédé selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide dodécanedioïque doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide dodécanedioïque doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est choisi dans le groupe constitué du dodecanedioate de dioctyldodecyle (CAS 129423-55-8), du dodecanedioate de diisocetyle (CAS 131252-83-0) et leurs mélanges.

Dans un mode de réalisation, le procédé selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide azélaïque doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide azélaïque doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est choisi dans le groupe constitué de l'azélate de diméthyle (CAS 1732-10-1), de l'azélate de di(2-éthylhexyle) et leurs mélanges.

Dans un mode de réalisation, le procédé selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un

seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide glutarique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide glutarique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est choisi dans le groupe constitué du glutarate de diméthyle (CAS 1119-40-0), du glutarate de diisobutyle (CAS 71195-64-7), du glutarate de diisostéaryle, du 2-méthylglutarate de diméthyle (CAS 14035-94-0)et leurs mélanges.

Dans un mode de réalisation, le procédé selon l'invention est caractérisé en ce que ledit au moins un solvant est un acide sébacique doublement estérifié par deux alcools identiques ou différents, chacun des deux alcools étant un alcool linéaire ou ramifié, ou un mélange d'isomères dans n'importe quelles proportions le cas échéant, ou bien un seul des isomères le cas échéant, l'alcool étant en $C_1$-$C_{30}$, préférentiellement en $C_1$-$C_{20}$, préférentiellement en $C_1$-$C_{10}$.

Dans un mode de réalisation, le procédé selon l'invention est caractérisé en ce que ledit au moins un solvant est un acide malonique doublement estérifié par deux alcools identiques ou différents, chacun des deux alcools étant choisi dans le groupe constitué par le méthanol, l'éthanol, les propanols de formule brute $C_3H_8O$, les butanols de formule brute $C_4H_{10}O$, les pentanols de formule brute $C_5H_{12}O$, les hexanols de formule brute $C_6H_{14}O$, les heptanols de formule brute $C_7H_{16}O$, les octanols de formule brute $C_8H_{18}O$, les nonanols de formule brute $C_9H_{20}O$, les décanols de formule brute $C_{10}H_{22}O$, les undécanols de formule brute $C_{11}H_{24}O$, les dodécanols de formule brute $C_{12}H_{26}O$, les tridécanols de formule brute $C_{13}H_{28}O$, les tétradécanols de formule brute $C_{14}H_{30}O$, les pentadécanols de formule brute $C_{15}H_{32}O$, les hexadécanols de formule brute $C_{16}H_{34}O$, les heptadécanols de formule brute $C_{17}H_{36}O$, les octadécanols de formule brute $C_{18}H_{38}O$, les nonadécanols de formule brute $C_{19}H_{40}O$ et les eicosanols de formule brute $C_{20}H_{42}O$.

Dans un mode de réalisation, le procédé selon l'invention est caractérisé en ce que ledit au moins un solvant est un acide malonique doublement estérifié par deux alcools identiques ou différents, chacun des deux alcools étant choisi dans le groupe constitué du méthanol (CAS 67-56-1), de l'éthanol (CAS 64-17-5), du propan-1-ol (ou n-propanol) (CAS 71-23-8), du propan-2-ol (ou isopropanol) (CAS 67-63-0), du butan-1-ol (ou n-butanol) (CAS 71-36-3), du (R)-butan-2-ol (CAS 14898-79-4), du (S)-butan-2-ol (CAS 4221-99-2), du 2-méthylpropan-1-ol (CAS 78-83-1), du 2-méthylpropan-2-ol (CAS 75-65-0), du 2-méthylpropan-2-ol (CAS 75-65-0), du pentan-1-ol (CAS 71-41-0), du 3-méthylbutan-1-ol (CAS 123-51-3), du 2-méthylbutan-1-ol (CAS 137-32-6), du 2,2-diméthylpropan-1-ol (CAS 75-84-3), du pentan-3-ol (CAS 584-02-1), du pentan-2-ol (CAS 6032-29-7), du 3-méthylbutan-2-ol (CAS 598-75-4), du 2-méthylbutan-2-ol (CAS 75-85-4), de l'hexan-1-ol (CAS 111-27-3), de l'heptan-1-ol (CAS 111-70-6), du dodécan-1-ol (CAS 112-53-8), de l'octan-1-ol (CAS 111-87-5), du 2-éthylhexan-1-ol (CAS 104-76-7), de l'octadécan-1-ol (CAS 112-92-5), du décan-1-ol (CAS 112-30-1) et du dodécan-1-ol (CAS 112-53-8).

Dans un mode de réalisation, le procédé selon l'invention est caractérisé en ce que ledit au moins un solvant est le malonate de diéthyle (CAS 105-53-3).

L'invention concerne également une formulation cosmétique, caractérisée en ce qu'elle comprend :

- au moins un ingrédient cosmétique selon l'invention tel que décrit ci-dessus ; et
- au moins un véhicule cosmétiquement acceptable.

La formulation cosmétique selon l'invention peut en outre comprendre un ou plusieurs filtres solaires complémentaires organiques ou minéraux.

Parmi les filtres organiques actifs dans l'UV-A et/ou l'UV-B, hydrophiles ou lipophiles, on citera à titre d'exemples,

les dérivés cinnamiques, les dérivés salicyliques, les dérivés du camphre, les dérivés de la triazine, les dérivés de la benzophénone, les dérivés du dibenzoylméthane, les dérivés de $\beta$,$\beta$-diphénylacrylate, les dérivés de l'acide p-aminobenzoïque, les filtres polymères et les filtres silicones.

Parmi les filtres minéraux, on citera à titre d'exemples, des agents photoprotecteurs agissant par blocage physique (réflexion et/ou diffusion) du rayonnement UV que sont des pigments ou bien encore des nanopigments dont la taille moyenne des particules primaires est comprise entre 5 nm et 500 nm, de préférence entre 100 et 250 nm. Ces pigments ou nanopigments sont des oxydes métalliques enrobés ou non, par exemple des nanopigments d'oxyde de titane, d'oxyde de fer, d'oxyde de zinc, d'oxyde de zirconium ou d'oxyde de cérium. Lorsqu'ils sont enrobés, les agents d'enrobage sont par exemple l'alumine et/ou le stéarate d'aluminium.

La formulation cosmétique selon l'invention peut également comprendre des dérivés de la tyrosine ou de la dihydroxyacétone (DHA) qui sont des agents de bronzage et/ou de brunissage artificiels de la peau (agents autobronzants).

La formulation cosmétique selon l'invention peut comprendre en outre des adjuvants cosmétiques classiques utilisés comme supports et constituants de formulation notamment choisis parmi les corps gras, les solvants organiques, les épaississants ioniques ou non ioniques, les adoucissants, les opacifiants, les émollients, les silicones, les agents anti-mousse, les tensioactifs, les charges, les séquestrants, les polymères, les propulseurs, les agents alcalinisants ou acidifiants, les colorants, ou tout autre ingrédient habituellement utilisé en cosmétique.

Les corps gras peuvent être constitués par une huile ou une cire ou leurs mélanges, et ils comprennent également des acides gras, des alcools gras et des esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-$\alpha$-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthylcellulose.

La formulation cosmétique selon l'invention peut comprendre en outre des adjuvants cosmétiques classiques utilisés comme actifs à savoir conservateurs ou stabilisants comme les antioxydants ou les stabilisants.

Elle peut en outre comprendre des agents hydratants, des vitamines ou des parfums.

La formulation cosmétique selon l'invention, peut se présenter en particulier sous forme d'émulsion, simple ou complexe (H/E, E/H, H/E/H ou E/H/E) telle qu'une crème, un lait, un gel ou un gel crème, et être préparée selon les techniques bien connues de l'homme de l'art, en particulier celles destinées à la préparation d'émulsions de type huile-dans-eau (H/E) ou eau-dans-huile (E/H).

Elle peut éventuellement être conditionnée en aérosol et se présenter sous forme de mousse ou de spray.

La formulation cosmétique selon l'invention peut être utilisée comme composition protectrice de l'épiderme humain ou des cheveux contre les rayons ultraviolets, comme composition antisolaire ou comme produit de maquillage.

L'invention concerne également l'utilisation d'une formulation cosmétique selon l'invention telle que décrite ci-dessus, en tant que formulation cosmétique solaire.

L'invention concerne également l'utilisation d'une formulation cosmétique selon l'invention telle que décrite ci-dessus, en tant que formulation cosmétique anti-âge.

Exemples

Exemple 1 : détermination du pourcentage massique de la karanjine et du pongamol par chromatographie phase liquide

Dans tous les exemples suivants, les pourcentages massiques en pongamol et en karanjine sont mesurés par CLHP selon une méthode classique.

La colonne utilisée est une colonne Gemini NX-C18 de référence 00G-4454-50, Phenomenex dont les caractéristiques sont 250 x 4,6mm, 5$\mu$m, 110Å.

Les réactifs utilisés sont les suivants :

Tableau 1

| Réactifs | Numéro CAS | Fournisseur | Qualité, référence |
|---|---|---|---|
| Eau | 7732-18-5 | VWR | HiPerSolv |
| Acide formique | 64-18-6 | Aldrich | - |
| Acétonitrile | 75-05-8 | VWR | HiPerSolv |

(suite)

| Réactifs | Numéro CAS | Fournisseur | Qualité, référence |
|---|---|---|---|
| Karaniine | 521-88-0 | Chromadex | ASB-00011275-010 |
| Pongamol | 484-33-3 | Biosynthis | nd |
| Diméthylsulfoxide | 67-68-5 | Aldrich | - |

Les conditions analytiques sont les suivantes :

Tableau 2

| Paramètres | Consignes | | |
|---|---|---|---|
| Injecteur | Température ambiante | | |
| Détecteur | UV<br>À = 250 nm | | |
| Four | 25°C | | |
| Débit | 1,0 mL/min | | |
| Phase mobile | Pompe A : Eau acidifiée (0,1% d'acide formique)<br>Pompe B : Acétonitrile | | |
| Gradient d'élution linéaire | t (min)<br>0<br>42<br>45<br>50 | % EA<br>95<br>0<br>95<br>95 | % ACN<br>5<br>100<br>5<br>5 |
| Durée de l'analyse | 50 min | | |
| Volume injecté | 10 $\mu$L | | |

La calibration a été effectuée à partir d'une solution mère comprenant de 14,4mg de standard de karanjine brute (p=95,7%) et 33,4mg de pongamol brut (p=90,0%) 50mL de DMSO.
La préparation de la gamme a été effectuée avec du DMSO.
Les temps de rétention sont les suivants :

Tableau 3

| Composés | Temps de rétention |
|---|---|
| Karanjine | 28,8 min |
| Pongamol | 33,4 min |

Le chromatogramme d'une solution de calibration est donné en Figure 1.
Le chromatogramme d'un échantillon d'huile de karanja est donné en Figure 2.
Le logiciel LC Solutions® permet de déterminer la concentration (mg/mL) en karanjine et pongamol.

Exemple 2 : procédé selon l'invention

Une huile de karanja ayant subi une démucilagination et une désodorisation ayant les concentrations suivantes en pongamol et karanjine est utilisée :

Tableau 4

| | |
|---|---|
| Indice d'acide (mg KOH/g) | 6 |
| Pourcentage massique pongamol % | 1,00 |

(suite)

| | |
|---|---|
| Pourcentage massique karanjine (%) | 2,5 |
| Rapport pongamol/karanjine | 040 |

Etape 1 : distillation moléculaire

Une étape de distillation moléculaire est réalisée, sur un appareil KDL5 avec les conditions suivantes : température de distillation 170°C, vide $10^{-2}$ mbar, alimentation fixée à 250mL/h.
Le bilan massique de la distillation est donné ci-après :

Tableau 5

| | |
|---|---|
| Phase lourde (%) | 91 |
| Phase légère (%) | 9 |

La phase légère de distillation est ensuite caractérisée :

Tableau 6

| | |
|---|---|
| Indice d'acide (mg KOH/g) | 70,0 |
| Pourcentage massique pongamol (%) | 10,45 |
| Pourcentage massique karanjine (%) | 14,8 |
| Rapport $m_{pongamol}/m_{karanjine}$ | 0,71 |

Etape 2 : précipitation de la karanjine

A l'huile de karanja ayant subi la distillation moléculaire (la phase légère de distillation) est ajoutée une même masse de sébacate de diéthyle (CAS 110-40-7) (proportions 50/50).
Le mélange est agité 24h à 15°C. Un précipité est formé. La suspension obtenue est ensuite filtrée sur filtre 11 $\mu$m.
Le bilan de la précipitation est donné ci-après :

Tableau 7

| | |
|---|---|
| Phase légère (surnageant) (%) | 89,7 |
| Phase lourde (précipité) % | 10,3 |

Les teneurs en pongamol et karanjine dans les phases légère sous forme de solution et lourde sous forme de précipité sont ensuite mesurées :

Tableau 8

| | |
|---|---|
| Pourcentage massique pongamol phase légère (surnageant) (%) | 5,58 |
| Pourcentage massique pongamol phase lourde (précipité) (%) | 1,79 |
| Pourcentage massique karanjine phase légère (surnageant) (%) | 3,18 |
| Pourcentage massique karanjine phase lourde (précipité) (%) | 40,1 |
| Rapport $m_{pongamol}/m_{karanjine}$ phase légère (surnageant) | 1,75 |
| Rapport $m_{pongamol}/m_{karanjine}$ phase lourde (précipité) | 0,04 |

La phase légère ou surnageant est une solution composée d'environ 50% de sébacate de diéthyle (CAS 110-40-7), et d'environ 50% d'extrait d'huile de karanja enrichi en pongamol.
Les rendements d'extraction sont donnés ci-après :

Tableau 9

| | |
|---|---|
| Rendement extraction pongamol phase légère (surnageant) (%) | 95,7 |
| Rendement extraction pongamol phase lourde (précipité) (%) | 4,3 |
| Rendement extraction karanjine phase légère (surnageant) (%) | 38,5 |
| Rendement extraction karanjine phase lourde (précipité) (%) | 61,5 |

En conclusion, avec une utilisation de sébacate de diéthyle (CAS 110-40-7) à 50/50, on obtient :

- une élimination de 61,5% de la karanjine de la phase légère ou surnageant sous forme de solution ;
- une évolution du rapport $m_{pongamol}/m_{karanjine}$ d'un facteur 2,46 (1,75/0,71) ;
- un rendement d'extraction du pongamol de 95,7 %.

La phase légère ou surnageant sous forme de solution, une fois séparée de la phase lourde sous forme de précipité, est un ingrédient cosmétique selon l'invention consistant en une solution comprenant un extrait d'huile de karanja et du sébacate de diéthyle (CAS 110-40-7). Cet ingrédient cosmétique/cette solution pourra par exemple être incorporé(e) directement dans une formulation cosmétique, sans élimination du sébacate de diéthyle (CAS 110-40-7) et sans étape de solubilisation du pongamol.
Cet ingrédient cosmétique consistant en une solution est caractérisé comme suit :

Tableau 10

| Caractéristique | Résultat |
|---|---|
| Apparence | Huile rouqe/orange |
| Couleur (échelle Gardner) | <16 |
| Indice de réfraction (25°C) | 1,504 |
| Densité (20°C ; g/cm$^3$) | 1,034 |
| Indice d'acide (mg KOH/g) | 36,1 |
| Pourcentage massique pongamol (%) | 5,58 |
| Pourcentage massique karanjine (%) | 3,18 |
| Humidité (K.F, %) | <0,1 |

Exemple 3 : tests de précipitation avec d'autres solvants selon l'invention

Une distillation moléculaire d'une huile de karanja provenant d'un autre lot que celui utilisé dans l'exemple 1 est réalisée au moyen d'un distillateur couche mince industriel, aux conditions suivantes : température de distillation 170°C, vide $10^{-2}$ mbar. L'huile de karanja avant subi la distillation moléculaire est caractérisée comme suit :

Tableau 11

| | |
|---|---|
| Indice d'acide (mg KOH/g) | 67,0 |
| Pourcentage massique pongamol (%) | 2,65 |
| Pourcentage massique karanjine (%) | 15,5 |
| Rapport $m_{pongamol}/m_{karanjine}$ | 0,17 |

Des essais de précipitation sont ensuite effectués sur l'huile de karanja avec différents solvants :

- le sébacate de diéthyle (CAS 110-40-7) (essai 1) ;

- le sébacate de diisopropyle (CAS 7491-02-3) (essai 2).

Le mélange est agité 24h à 15°C. Un précipité est formé. La suspension obtenue est ensuite filtrée sur filtre 11 μm.

Les bilans massiques des essais sont donnés ci-après :

Tableau 12

| Essai | 1 | 2 |
|---|---|---|
| Nature solvant | sébacate de diéthyle (CAS 110-40-7) | sébacate de diisopropyle (CAS 7491-02-3) |
| Pourcentage massique solvant/huile de karanja | 20/80 | 20/80 |
| Phase légère (surnageant) (%) | 53,7 | 56,0 |
| Phase lourde (précipité) (%) | 46,3 | 44,0 |

Les bilans massiques sont similaires.

Les teneurs en pongamol et karanjine dans les phases légères sous forme de solution et lourde sous forme de précipité sont ensuite mesurées :

Tableau 13

| Essai | 1 | 2 |
|---|---|---|
| Nature solvant | sébacate de diéthyle (CAS 110-40-7) | sébacate de diisopropyle (CAS 7491-02-3) |
| Pourcentage massique solvant/huile de karanja | 20/80 | 20/80 |
| Pourcentage massique pongamol phase légère (surnageant) (%) | 2,67 | 2,51 |
| Pourcentage massique pongamol phase lourde (précipité) (%) | 2,15 | 2,51 |
| Pourcentage massique karanjine phase légère (surnageant) (%) | 0,72 | 4,19 |
| Pourcentage massique karanjine phase lourde (précipité) (%) | 25,22 | 14,90 |
| Rapport $m_{pongamol}/m_{karanjine}$ phase légère (surnageant) | 3,70 | 0,60 |

Les rendements d'extraction sont donnés ci-après :

Tableau 14

| Essai | 1 | 2 |
|---|---|---|
| Nature solvant | sébacate de diéthyle (CAS 110-40-7) | sébacate de diisopropyle (CAS 7491-02-3) |
| Pourcentage massique solvant/huile de karanja | 20/80 | 20/80 |
| Rendement extraction pongamol phase légère (surnageant) (%) | 65,1 | 63,8 |
| Rendement extraction pongamol phase lourde (précipité) (%) | 34,9 | 36,2 |
| Rendement extraction karanjine phase légère (surnageant) (%) | 3,0 | 18,2 |

(suite)

| Essai | 1 | 2 |
|---|---|---|
| Rendement extraction karanjine phase lourde (précipité) (%) | 97,0 | 81,8 |

En conclusion, avec une utilisation du sébacate de diéthyle (CAS 110-40-7) à 20/80, on obtient :

- une élimination de 97,0% de la karanjine de la phase légère ou surnageant sous forme de solution ;
- une évolution du rapport $m_{pongamol}/m_{karanjine}$ d'un facteur 21,76 (3,70/0,17) ;
- un rendement d'extraction du pongamol de 65,1 %.

En conclusion, avec une utilisation du sébacate de diisopropyle (CAS 7491-02-3) à 20/80, on obtient :

- une élimination de 81,8% de la karanjine de la phase légère ou surnageant sous forme de solution ;
- une évolution du rapport $m_{pongamol}/m_{karanjine}$ d'un facteur 3,53 (0,6/0,17) ;
- un rendement d'extraction du pongamol de 63,8 %.

Le sébacate de diéthyle est donc supérieur au sébacate de diisopropyle en ce qui concerne sa capacité à précipiter la karanjine, le rendement d'extraction du pongamol étant similaire.

Ici encore, la phase légère ou surnageant sous forme de solution, une fois séparée de la phase lourde sous forme de précipité, est un ingrédient cosmétique selon l'invention consistant en une solution comprenant un extrait d'huile de karanja et un solvant (sébacate de diéthyle ou sébacate de diisopropyle). Cet ingrédient cosmétique/cette solution pourra par exemple être incorporé directement dans une formulation cosmétique, sans élimination du solvant (sébacate de diéthyle ou sébacate de diisopropyle) et sans étape de solubilisation du pongamol.

Exemple 4 : tests de précipitation avec différentes proportions de sébacate de diéthyle

Une distillation moléculaire est réalisée dans les mêmes conditions que celles décrites à l'exemple 1, à partir d'un autre lot d'huile de karanja. A l'issue de la distillation, moléculaire, l'huile de karanja est caractérisée comme suit :

Tableau 15

| | |
|---|---|
| Indice d'acide (mg KOH/g) | 67,0 |
| Pourcentage massique pongamol (%) | 3,25 |
| Pourcentage massique karanjine (%) | 11,4 |
| Rapport $m_{pongamol}/m_{karanjine}$ | 0,28 |

Des essais 1 à 8 de précipitation sont ensuite effectués sur l'huile de karanja avec le sébacate de diéthyle (CAS 110-40-7), selon différentes proportions sébacate de diéthyle/huile de karanja.

Les phases légère ou surnageant sous forme de solution et lourde ou précipité sont séparées par centrifugation.

Les bilans massiques des essais sont donnés ci-après:

Tableau 16

| Essai | 1 | 2 | 3 | 4 | 5 | 6 | 7 | 8 |
|---|---|---|---|---|---|---|---|---|
| % massique sébacate de diéthyle/huile de karanja | 84/16 | 80/20 | 71,5/28,5 | 62,5/37,5 | 50/50 | 40/60 | 25/75 | 20/80 |
| Phase légère (surnageant) (%) | 95,6 | 97,3 | 94,5 | 90,6 | 84,5 | 75,1 | 61,6 | 63,1 |
| Phase lourde (précipité) (%) | 4,4 | 2,7 | 5,5 | 9,4 | 15,5 | 24,9 | 38,4 | 36,9 |

Les teneurs en pongamol et karanjine dans les phases légère (surnageant) et lourde (précipité) sont ensuite mesurées :

Tableau 17

| Essai | 1 | 2 | 3 | 4 | 5 | 6 | 7 | 8 |
|---|---|---|---|---|---|---|---|---|
| % massique sébacate de diéthyle/huile de karanja | 84/16 | 80/20 | 71,5/28,5 | 62,5/37,5 | 50/50 | 40/60 | 25/75 | 20/80 |
| Pourcentage massique pongamol phase légère (surnageant) (%) | 0,57 | 0,64 | 0,87 | 1,35 | 1,48 | 1,99 | 2,44 | 2,51 |
| Pourcentage massique pongamol plase lourde (précipité) (%) | 0,68 | 0,67 | 0,80 | 1,01 | 1,43 | 1,71 | 2,24 | 2,41 |
| Pourcentage massique karanjine phase légère (surnageant) (%) | 1,50 | 1,64 | 2,18 | 2,40 | 2,50 | 2,62 | 2,85 | 2,91 |
| Pourcentage massique karanjine phase lourde (précipité) (%) | 13,42 | 8,91 | 23,74 | 19,28 | 21,67 | 17,39 | 14,80 | 15,20 |
| Rapport $m_{pongamol}/m_{karanjine}$ phase légère (surnageant) (%) | 0,38 | 0,39 | 0,40 | 0,56 | 0,59 | 0,76 | 0,86 | 0,86 |

Les rendements d'extraction sont donnés ci-arès :

Tableau 18

| Essai | 1 | 2 | 3 | 4 | 5 | 6 | 7 | 8 |
|---|---|---|---|---|---|---|---|---|
| % massique sébacate de diéthyle/huile de karanja | 84/16 | 80/20 | 71,5/28,5 | 62,5/37,5 | 50/50 | 40/60 | 25/75 | 20/80 |
| Rendement extraction pongamol phase légère (surnageant) (%) | 91,1 | 97,8 | 90,2 | 98,2 | 76,8 | 76,9 | 60,9 | 60,9 |
| Rendement extraction pongamol phase lourde (précipité) (%) | 8,9 | 2,2 | 9,8 | 1,8 | 23,2 | 23,1 | 39,1 | 39,1 |
| Rendement extraction karanjine phase légère (surnageant) (%) | 72,5 | 72,0 | 64,0 | 49,8 | 36,9 | 28,8 | 20,3 | 20,1 |
| Rendement extraction karanjine phase lourde (précipité) (%) | 27,5 | 28,0 | 36,0 | 50,2 | 63,1 | 71,2 | 79,7 | 79,1 |

Une courbe représentant les rendements d'extraction du pongamol et de la karanjine dans la phase légère ou surnageant sous forme de solution en fonction de la proportion de sébacate de diéthyle utilisée est donnée en Figure 3. La Figure 3 montre l'évolution des rendements d'extraction en pongamol et en karanjine en fonction de la proportion de sébacate de diéthyle utilisée.

En fonction des pourcentages massiques en pongamol et karanjine, la proportion de solvant doit être ajustée pour obtenir un pourcentage massique en karanjine conforme au cahier des charges et obtenir un rapport $m_{pongamol}/m_{karanjine}$ supérieur ou égal à 0,5.

Ici encore, en particulier pour les essais 4-8, la phase légère ou surnageant sous forme de solution, une fois séparée de la phase lourde ou précipité, est un ingrédient cosmétique selon l'invention consistant en une solution comprenant un extrait d'huile de karanja et du sébacate de diéthyle. Cet ingrédient cosmétique/cette solution pourra par exemple être incorporé directement dans une formulation cosmétique, sans retrait du sébacate de diéthyle et sans étape de solubilisation du pongamol.

Exemple 5 : exemple de formulation selon l'invention

Un exemple de formulation selon l'invention, ainsi q'un exemple de mode opératoire est donné ci-après :

Tableau 19

| Ingrédient | % en poids | Fournisseur |
|---|---|---|
| **Phase A** | | |
| Phase légère ou surnageant sous forme de solution = ingrédient cosmétique selon l'invention (solvant : sébacate de diéthyle) | 7,5 | Biosynthis |
| Salicylate d'éthylhexyle | 5,0 | Svmrise |
| Octocrylène (CAS 6197-30-4) | 2,0 | DSM |
| Avobenzone (CAS 70356-09-1) | 2,0 | DSM |
| | | |
| **Phase B** | | |
| Caprylyl Méthicone (CAS 17955-883) | 4,0 | Dow Corning |
| Coconut alkanes | 3,0 | Biosynthis |
| Triméthylsiloxysilicate (et) Polypropylsilsesquioxane | 3,0 | Dow Corning |
| PEG-12 Diméthicone (CAS 68937-54-2) | 4,0 | Dow Corning |
| | | |
| **Phase C** | | |
| Décyl Glucoside | 0,5 | BASF |
| Glycérine | 5,0 | Oléon |
| Eau déionisée | 61,6 | / |
| | | |
| **Phase D** | | |
| Polyacrylamide (et) C13-14 Isoparaffine (et) Laureth-7 | 2,0 | Seppic |
| | | |
| **Phase E** | | |
| Butyl parabène (CAS 94-26-8) | 0,4 | Pharmco-AAPER |

La formulation cométique est préparée selon le mode opératoire suivant qui comporte 9 étapes :

1) Dans un mélangeur séparé, mélanger l'ensemble des composants de la phase A, sous agitation à 60°C et jusqu'à homogénéisation complète ;
2) Refroidir jusqu'à température ambiante sous agitation constante ;
3) En parallèle, mélanger dans un second mélangeur, les constituants de la phase B jusqu'à homogénéisation complète ;
4) Additionner à température ambiante les phases A et B, jusqu'à homogénéisation complète ;
5) Mélanger dans un troisième mélangeur les constituants de la phase C ;
6) Mélanger les phases (A + B) et C sous agitation ;
7) Agiter pendant 20 minutes à 1500 tours/minute ;
8) Mélanger (A + B + C) + D, jusqu'à homogénéisation complète ;
9) Ajouter E à (A + B + C + D) en maintenant l'agitation.

Exemple 6 : Propriétés protectrices d'une formulation cosmétique selon l'invention (in vivo)

Un ingrédient cosmétique selon l'invention est réalisé selon la méthode présentée en exemple 2, cet ingrédient

cosmétique comprend environ 50% de sébacate de diéthyle (fournis par ARKEMA) et environ 50% d'extrait d'huile de karanja enrichi en pongamol.

L'ingrédient cosmétique selon l'invention est ensuite formulé, la formulation cosmétique selon l'invention présente les compositions qualitatives et quantitatives suivantes :

Tableau 20

| **Composants** | **% en poids** | **Fournisseur** |
|---|---|---|
| ingrédient cosmétique selon l'invention (solvant : sébacate de diéthyle, environ 50%) | 65 | BIOSYNTHIS |
| Coconut oil gel 35 (INCI : Coconut oil and dilinoleic acid / Propanediol/ Octyldodecanol copolymer) | 30 | BIOSYNTHIS |
| Trihydroxystéarine (CAS 139-44-6) | 5 | BASF/Cognis |

Le Facteur de Protection Solaire (FPS) a été déterminé pour la formulation cosmétique selon l'invention, selon les recommandations de la Norme Internationale ISO 24444 (Novembre 2010) sur un panel de trois sujets.

Les résultats obtenus sont présentés dans le tableau ci-dessous :

Tableau 21

| Sujet | Age | ITA° | Phototype | DEM np | DEM p | FPSi formulation testée | FPSi produit standard P2 |
|---|---|---|---|---|---|---|---|
| Sujet 1 | 54 | 50,7 | II | 31,1 | 1642,6 | 52,9 | 16,0 |
| Sujet 2 | 26 | 411 | III | 32,0 | 1946,7 | 60,8 | 16,0 |
| Sujet 3 | 48 | 52,6 | II | 26,0 | 1581,7 | 60,8 | 16,0 |
| Moyenne | | | | | | 58,2 | 16,0 |
| Ecart type | | | | | | 4,6 | 0,0 |
| DEMnp : Dose Erythémale Minimale de la peau non protégée<br>DEMp : Dose Erythémale Minimale de la peau protégée<br>FPSi : Facteur de Protection Solaire individuel<br>ITA : Individual Typology Angle | | | | | | | |

La formulation cosmétique selon l'invention présente un FPS moyen égal à 58,2, ce qui correspond à une protection solaire forte.

Les bonnes propriétés solaires de la formulation cosmétique selon l'invention sont donc confirmées in vivo.

Exemple 7 : Propriétés protectrices d'un ingrédient cosmétique selon l'invention (in vitro)

Un ingrédient cosmétique selon l'invention est réalisé selon la méthode présentée en exemple 2, cet ingrédient cosmétique comprend environ 50% de sébacate de diéthyle et environ 50% d'extrait d'huile de karanja enrichi en pongamol.

Le Facteur de Protection Solaire a été déterminé, pour l'ingrédient cosmétique selon l'invention et pour une huile de karanja désodorisée, au moyen de plaques en PMMA selon la méthode décrite dans la publication Pissavini et al., « Détermination of the in vitro FPS », Cosmetics and toiletries, 2003, vol. 118, no10, pp. 63-72.

Les résultats obtenus sont présentés dans le tableau ci-dessous :

Tableau 22

| Composition | Pourcentage massique de pongamol (%) | FPS / FPS IR | Protection UVA / Protection UVA IR |
|---|---|---|---|
| Huile de Karanja désodorisée | 1,4 | 182 / 12,7 | 9,9 / 7,8 |
| Ingrédient cosmétique selon l'invention | 7,7 | 190 / 174 | 76 / 72 |

(suite)

| Composition | Pourcentage massique de pongamol (%) | FPS / FPS IR | Protection UVA / Protection UVA IR |
|---|---|---|---|
| Témoin PMMA | NA | 25,7 | 9,0 |
| FPS : Facteur de Protection Solaire<br>IR : après irradiation<br>FPS IR : Facteur de Protection Solaire après irradiation | | | |

L'ingrédient cosmétique selon l'invention présente un FPS très élevé par rapport à celui de l'huile de karanja désodorisée.

Les bonnes propriétés solaires de l'ingrédient cosmétique selon l'invention sont donc confirmées in vitro.

On remarque que l'ingrédient cosmétique selon l'invention présente un FPS augmenté d'un facteur de plus de 10, et une protection UVA également augmentée d'un facteur proche de 10.

Egalement, l'irradiation de l'ingrédient cosmétique selon l'invention a relativement peu d'influence sur le FPS (de 190 à 174, le FPS est diminué d'environ 9%) et sur la protection UVA (de 76 à 72, la protection UVA est diminuée d'environ 5%), tandis que l'irradiation de l'huile de karanja désodorisée a beaucoup d'influence sur le FPS (de 18,2 à 12,7, le FPS est diminué d'environ 30%) et sur la protection UVA (de 9,9 à 7,8, la protection UVA est diminuée d'environ 20%).

## Revendications

1. Ingrédient cosmétique, **caractérisé en ce qu'**il consiste en une solution d'au moins un extrait d'huile de karanja comprenant du pongamol (CAS 484-33-3) et de la karanjine (CAS 521-88-0) dans au moins un solvant choisi dans le groupe des composés de formule (I) suivante, ou leurs mélanges :

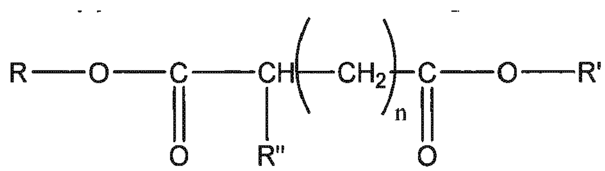

Formule (I)

dans laquelle :

- n est compris entre 0 et 19 ;
- R et R' identiques ou différents sont des alkyls provenant d'une estérification par un alcool linéaire ou ramifié de formule brute $C_xH_{2x+2}O$, x étant compris entre 1 et 30, préférentiellement entre 1 et 20, préférentiellement entre 1 et 10.
- R" est soit un atome d'hydrogène soit un groupement alkyle en $C_1$-$C_3$.

2. Ingrédient cosmétique selon la revendication 1, **caractérisé en ce que** ledit au moins un solvant est choisi dans le groupe constitué des sébacates (n=7), des adipates (n=3), des succinates (n=1), des dodécanedioates (n=9), des azélates (n=6), des glutarates (n=2), des malonates (n=0), et de leurs mélanges.

3. Ingrédient cosmétique selon l'une quelconque des revendications précédentes, **caractérisé en ce que** ledit au moins un solvant est choisi dans le groupe constitué des sebacates (n=7).

4. Ingrédient cosmétique selon l'une quelconque des revendications précédentes, **caractérisé en ce que** ledit au moins un solvant est le sébacate de diéthyle (CAS 110-40-7).

5. Ingrédient cosmétique selon l'une quelconque des revendications précédentes, **caractérisé en ce que** le pourcentage massique en ledit au moins un solvant dans ladite solution est compris entre 20% et 60%.

6. Ingrédient cosmétique selon l'une quelconque des revendications précédentes, **caractérisé en ce que** le pourcentage massique en pongamol dans ladite solution est supérieur à 1,30%.

7. Ingrédient cosmétique selon l'une quelconque des revendications précédentes, **caractérisé en ce que** le pourcentage massique en karanjine dans ladite solution est inférieur à 6%.

8. Ingrédient cosmétique selon l'une quelconque des revendications précédentes, **caractérisé en ce que** le rapport $m_{pongamol}/m_{karanjine}$ dans ladite solution est supérieur à 0,5.

9. Formulation cosmétique, **caractérisée en ce qu'**elle comprend :

   - au moins un ingrédient cosmétique selon l'une quelconque des revendications précédentes ; et
   - au moins un véhicule cosmétiquement acceptable.

10. Utilisation d'une formulation cosmétique selon la revendication 9, en tant que formulation cosmétique solaire et/ou en tant que formulation cosmétique anti-âge.

11. Procédé de précipitation sélective du pongamol (CAS 484-33-3) dans une huile de karanja comprenant du pongamol (CAS 484-33-3) et de la karanjine (CAS 521-88-0), **caractérisé en ce qu'**il comprend :

   1) au moins une étape d'addition à ladite au moins une huile de karanja d'au moins un solvant choisi dans le groupe des diesters de formule (I) suivante, ou leurs mélanges :

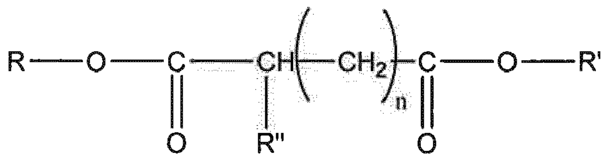

Formule (I)

   dans laquelle :

   - n est compris entre 0 et 19 ;
   - R et R' identiques ou différents sont des alkyls provenant d'une estérification par un alcool linéaire ou ramifié de formule brute $C_xH_{2x+2}O$, x étant compris entre 1 et 30, préférentiellement entre 1 et 20, préférentiellement entre 1 et 10.
   - R" est soit un atome d'hydrogène soit un groupement alkyle en $C_1$-$C_3$.

   ladite addition ayant pour effet de former une phase légère sous forme d'une solution et une phase lourde sous forme de précipité ; et
   2) au moins une étape de séparation des deux phases obtenues.

12. Procédé selon la revendication 11, **caractérisé en ce que** ledit au moins un solvant est choisi dans le groupe constitué des sébacates (n=7), des adipates (n=3), des succinates (n=1), des dodécanedioates (n=9), des azélates (n=6), des glutarates (n=2), des malonates (n=0), et de leurs mélanges.

13. Procédé selon l'une quelconque des revendications 11 à 12, **caractérisé en ce que** ledit au moins un solvant est choisi dans le groupe constitué des sébacates (n=7), de préférence le sébacate de diéthyle (CAS 110-40-7).

14. Procédé selon l'une quelconque des revendications 11 à 13, **caractérisé en ce que** lors de ladite étape 1), la proportion massique dudit au moins un solvant introduit dans ladite au moins une huile de karanja est comprise entre 60/40 et 20/80.

15. Procédé selon l'une quelconque des revendications 11 à 14, **caractérisé en ce qu'**il comprend en outre au moins une étape préalable de désodorisation de ladite au moins une huile de karanja et/ou au moins une étape préalable de distillation de ladite au moins une huile de karanja, lesdites étapes, lorsqu'elles sont toutes deux présentes, pouvant être effectuées dans n'importe quel ordre.

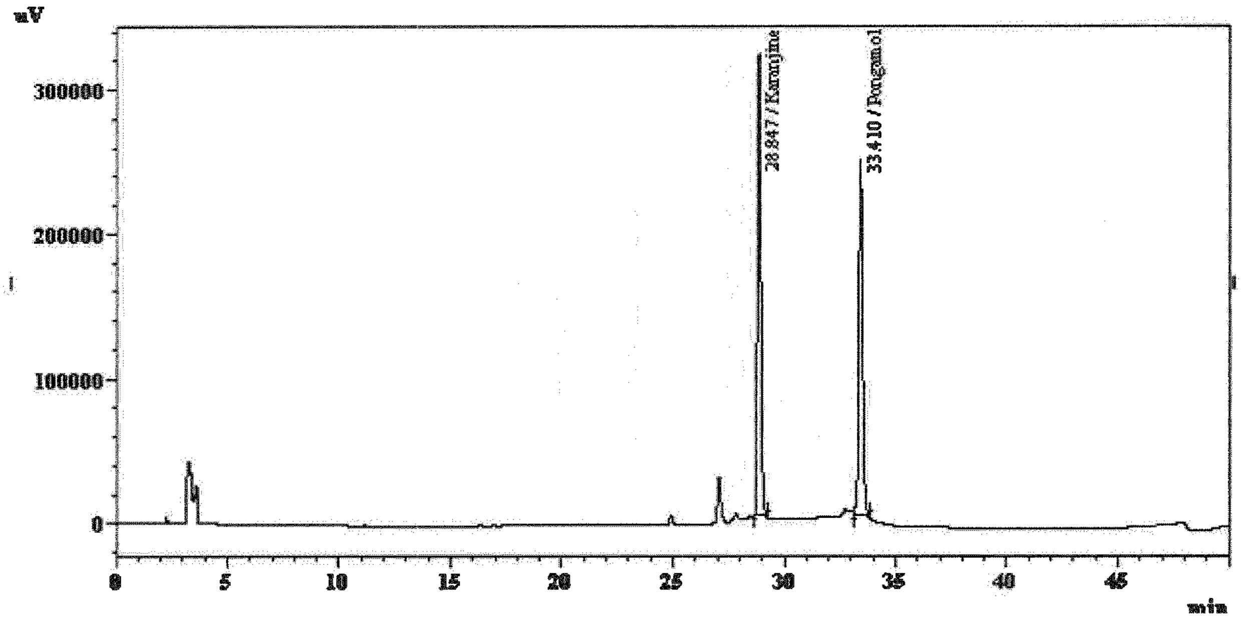
uV
300000
200000
100000
0
0
5
10
15
20
25
30
35
40
45
min
28.847 / Karanjine
33.410 / Pongamol

Figure 1

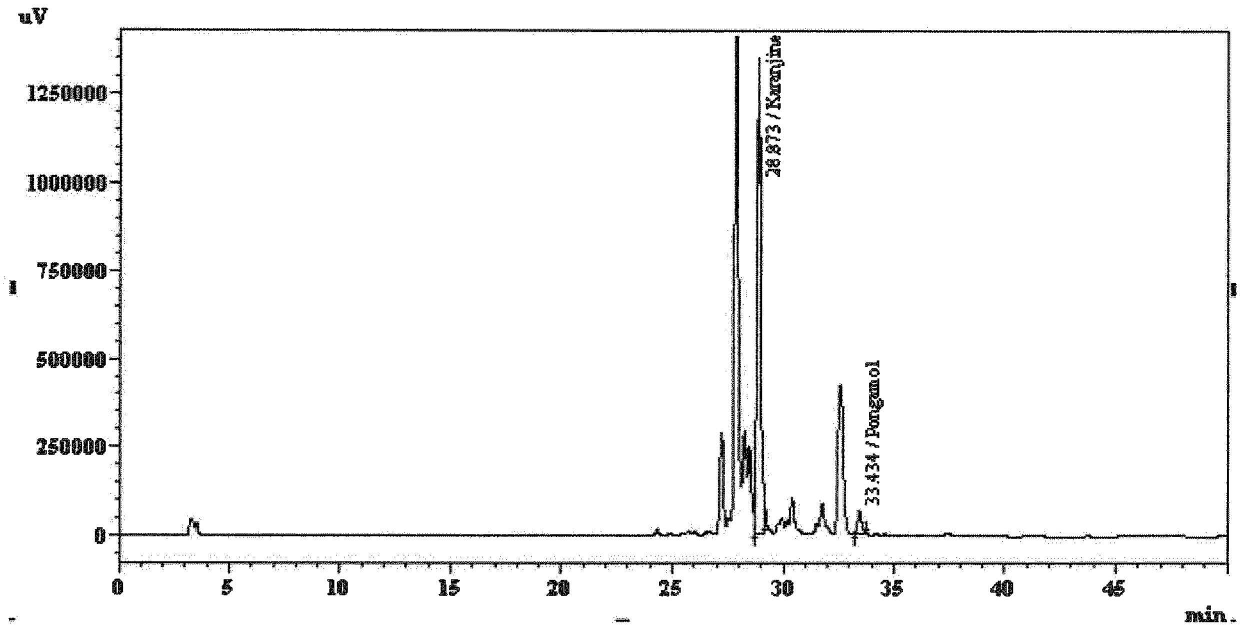

uV
1250000
1000000
750000
500000
250000
0
0
5
10
15
20
25
30
35
40
45
min.
28.873 / Karanjine
33.434 / Pongamol


Figure 2

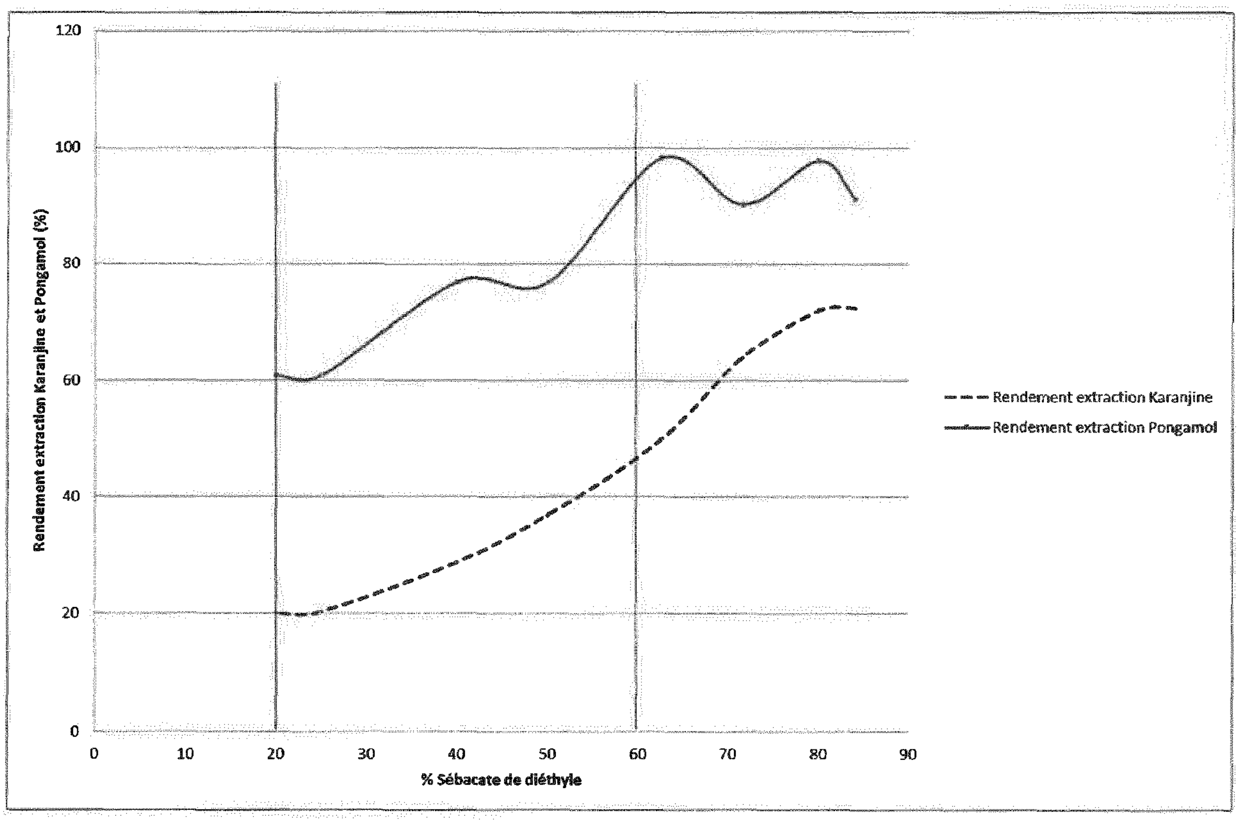
120
100
80
60
40
20
0
0
10
20
30
40
50
60
70
80
90
% Sébacate de diéthyle
Rendement extraction Karanjine et Pongamol (%)
Rendement extraction Karanjine
Rendement extraction Pongamol

**Figure 3**

Europäisches Patentamt
European Patent Office
Office européen des brevets

# RAPPORT DE RECHERCHE EUROPEENNE

Numéro de la demande
EP 19 19 6582

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | Nn: "Exhibitors Suppliers Day - USA", , 1 mai 2013 (2013-05-01), pages 1-16, XP055264364, Extrait de l'Internet: URL:http://www.youbuyfrance.com/medias/press/2013-03-05-cataloguefrench-pavilion-suppliers-day_7_5_2013_59_19.pdf [extrait le 2016-04-11] * page 4 * | 1-15 | INV. A61Q19/08 A61Q17/04 A61K8/9789 A61K8/37 A61K8/49 A61K36/486 |
| A | WO 2014/114888 A2 (THOREL JEAN-NOËL [FR]) 31 juillet 2014 (2014-07-31) * revendications 1-3,5 * * page 4, ligne 35 - page 5 * * pages 18-19; exemple 4 * | 1-15 | |
| A | "SPF 40 Sun Cream for the Face", GNPD; MINTEL, 1 septembre 2008 (2008-09-01), XP002716746, * product description; ingredients * | 1-15 | |
| | | | DOMAINES TECHNIQUES RECHERCHES (IPC) |
| A,D | WO 2014/016349 A1 (BIOSYNTHIS [FR]) 30 janvier 2014 (2014-01-30) * "transparent lipstick"; page 15, lignes 6-15 * | 1-15 | A61Q A61K |
| A | FR 2 720 643 A1 (CLARINS [FR]) 8 décembre 1995 (1995-12-08) * revendication 8 * * page 1, ligne 27 - page 2, ligne 3 * * page 5; exemples * | 1-15 | |

-/--

Le présent rapport a été établi pour toutes les revendications

| Lieu de la recherche | Date d'achèvement de la recherche | Examinateur |
|---|---|---|
| Munich | 14 novembre 2019 | Grillenberger, Sonja |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

2

EPO FORM 1503 03.82 (P04C02)

page 1 de 2

Europäisches Patentamt
European Patent Office
Office européen des brevets

# RAPPORT DE RECHERCHE EUROPEENNE

Numéro de la demande
EP 19 19 6582

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | FR 2 806 080 A1 (STEARINERIE DUBOIS FILS [FR]) 14 septembre 2001 (2001-09-14)<br>* revendications *<br>* exemples *<br>* page 1, lignes 30-35 *<br>----- | 1-15 | |
| A | EP 2 201 928 A2 (HENKEL AG & CO KGAA [DE]) 30 juin 2010 (2010-06-30)<br>* revendication 12 *<br>* SPF booster;<br>pages 76-79 *<br>----- | 1-15 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 14 novembre 2019 | Grillenberger, Sonja |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................................
& : membre de la même famille, document correspondant

2

EPO FORM 1503 03.82 (P04C02)

**ANNEXE AU RAPPORT DE RECHERCHE EUROPEENNE RELATIF A LA DEMANDE DE BREVET EUROPEEN NO.** EP 19 19 6582

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

14-11-2019

| Document brevet cité au rapport de recherche | Date de publication | Membre(s) de la famille de brevet(s) | Date de publication |
|---|---|---|---|
| WO 2014114888 A2 | 31-07-2014 | FR 3001150 A1 | 25-07-2014 |
| | | WO 2014114888 A2 | 31-07-2014 |
| WO 2014016349 A1 | 30-01-2014 | FR 2993777 A1 | 31-01-2014 |
| | | WO 2014016349 A1 | 30-01-2014 |
| FR 2720643 A1 | 08-12-1995 | AUCUN | |
| FR 2806080 A1 | 14-09-2001 | AT 275937 T | 15-10-2004 |
| | | AU 3937701 A | 24-09-2001 |
| | | DE 60105560 T2 | 13-10-2005 |
| | | EP 1263406 A1 | 11-12-2002 |
| | | ES 2228825 T3 | 16-04-2005 |
| | | FR 2806080 A1 | 14-09-2001 |
| | | US 2003031635 A1 | 13-02-2003 |
| | | WO 0168050 A1 | 20-09-2001 |
| EP 2201928 A2 | 30-06-2010 | DE 102008063306 A1 | 01-07-2010 |
| | | EP 2201928 A2 | 30-06-2010 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2014016349 A
- WO 2014114888 A
- FR 2720643
- FR 2762008
- GB 2237805 A
- WO 2014195639 A
- WO 9850005 A
- US 5152983 A


### Littérature non-brevet citée dans la description


- *CHEMICAL ABSTRACTS,* 482-33-3
- *CHEMICAL ABSTRACTS,* 521-88-0
- *CHEMICAL ABSTRACTS,* 70356-09-1
- *Exhibitors Suppliers Day - USA,* 01 Mai 2013, 1-16, http.//www.youbuyfrance.com/medias/press/2013-03-05-cataloguefrench-pavilion-suppliers-day 7 5 2013 59 19.pdf
- **GNP D.** FPS 40 Sun Cream for the Face. *MINTEL,* 01 Septembre 2008
- *CHEMICAL ABSTRACTS,* 484-33-3
- *CHEMICAL ABSTRACTS,* 111-20-6
- *CHEMICAL ABSTRACTS,* 67-56-1
- *CHEMICAL ABSTRACTS,* 64-17-5
- *CHEMICAL ABSTRACTS,* 71-23-8
- *CHEMICAL ABSTRACTS,* 67-63-0
- *CHEMICAL ABSTRACTS,* 71-36-3
- *CHEMICAL ABSTRACTS,* 14898-79-4
- *CHEMICAL ABSTRACTS,* 4221-99-2
- *CHEMICAL ABSTRACTS,* 78-83-1
- *CHEMICAL ABSTRACTS,* 75-65-0
- *CHEMICAL ABSTRACTS,* 71-41-0
- *CHEMICAL ABSTRACTS,* 123-51-3
- *CHEMICAL ABSTRACTS,* 137-32-6
- *CHEMICAL ABSTRACTS,* 75-84-3
- *CHEMICAL ABSTRACTS,* 584-02-1
- *CHEMICAL ABSTRACTS,* 6032-29-7
- *CHEMICAL ABSTRACTS,* 598-75-4
- *CHEMICAL ABSTRACTS,* 75-85-4
- *CHEMICAL ABSTRACTS,* 111-27-3
- *CHEMICAL ABSTRACTS,* 111-70-6
- *CHEMICAL ABSTRACTS,* 112-53-8
- *CHEMICAL ABSTRACTS,* 111-87-5

- *CHEMICAL ABSTRACTS,* 104-76-7
- *CHEMICAL ABSTRACTS,* 112-92-5
- *CHEMICAL ABSTRACTS,* 112-30-1
- *CHEMICAL ABSTRACTS,* 122-62-3
- *CHEMICAL ABSTRACTS,* 110-40-7
- *CHEMICAL ABSTRACTS,* 109-43-3
- *CHEMICAL ABSTRACTS,* 7491-02-3
- *CHEMICAL ABSTRACTS,* 124-04-9
- *CHEMICAL ABSTRACTS,* 2091-24-9
- *CHEMICAL ABSTRACTS,* 62479-36-1
- *CHEMICAL ABSTRACTS,* 108-63-4
- *CHEMICAL ABSTRACTS,* 16958-92-2
- *CHEMICAL ABSTRACTS,* 26720-21-8
- *CHEMICAL ABSTRACTS,* 6938-94-9
- *CHEMICAL ABSTRACTS,* 141-04-8
- *CHEMICAL ABSTRACTS,* 103-23-1
- *CHEMICAL ABSTRACTS,* 1330-86-5
- *CHEMICAL ABSTRACTS,* 33703-08-1
- *CHEMICAL ABSTRACTS,* 27178-16-1
- *CHEMICAL ABSTRACTS,* 141-28-6
- *CHEMICAL ABSTRACTS,* 627-93-0
- *CHEMICAL ABSTRACTS,* 57533-90-1
- *CHEMICAL ABSTRACTS,* 155613-91-5
- *CHEMICAL ABSTRACTS,* 106-19-4
- *CHEMICAL ABSTRACTS,* 85117-94-8
- *CHEMICAL ABSTRACTS,* 105-99-7
- *CHEMICAL ABSTRACTS,* 123-79-5
- *CHEMICAL ABSTRACTS,* 110-15-6
- *CHEMICAL ABSTRACTS,* 10595-82-1
- *CHEMICAL ABSTRACTS,* 106-65-0
- *CHEMICAL ABSTRACTS,* 123-25-1
- *CHEMICAL ABSTRACTS,* 14491-66-8
- *CHEMICAL ABSTRACTS,* 93280-98-9
- *CHEMICAL ABSTRACTS,* 925-06-4
- *CHEMICAL ABSTRACTS,* 2915-57-3
- *CHEMICAL ABSTRACTS,* 498-21-5
- *CHEMICAL ABSTRACTS,* 4676-51-1
- *CHEMICAL ABSTRACTS,* 204125-41-7
- *CHEMICAL ABSTRACTS,* 606491-29-6
- *CHEMICAL ABSTRACTS,* 56108-32-8
- *CHEMICAL ABSTRACTS,* 75906-62-6
- *CHEMICAL ABSTRACTS,* 878209-18-8
- *CHEMICAL ABSTRACTS,* 18447-89-7
- *CHEMICAL ABSTRACTS,* 204125-40-6
- *CHEMICAL ABSTRACTS,* 214280-22-5
- *CHEMICAL ABSTRACTS,* 57983-31-0
- *CHEMICAL ABSTRACTS,* 108763-17-3
- *CHEMICAL ABSTRACTS,* 56108-33-9
- *CHEMICAL ABSTRACTS,* 32774-96-2
- *CHEMICAL ABSTRACTS,* 51191-78-7
- *CHEMICAL ABSTRACTS,* 214280-27-0
- *CHEMICAL ABSTRACTS,* 131787-12-7
- *CHEMICAL ABSTRACTS,* 693-23-2
- *CHEMICAL ABSTRACTS,* 129423-55-8
- *CHEMICAL ABSTRACTS,* 131252-83-0
- *CHEMICAL ABSTRACTS,* 123-99-9
- *CHEMICAL ABSTRACTS,* 1732-10-1
- *CHEMICAL ABSTRACTS,* 110-94-1
- *CHEMICAL ABSTRACTS,* 1119-40-0
- *CHEMICAL ABSTRACTS,* 71195-64-7
- *CHEMICAL ABSTRACTS,* 14035-94-0
- *CHEMICAL ABSTRACTS,* 141-82-2
- *CHEMICAL ABSTRACTS,* 105-53-3
- *CHEMICAL ABSTRACTS,* 7732-18-5, 64-18-6, 75-05-8, 521-88-0, 484-33-3, 6768-5
- *CHEMICAL ABSTRACTS,* 6197-30-4
- *CHEMICAL ABSTRACTS,* 17955-883
- *CHEMICAL ABSTRACTS,* 68937-54-2
- *CHEMICAL ABSTRACTS,* 94-26-8
- *CHEMICAL ABSTRACTS,* 139-44-6

- **PISSAVINI et al.** Détermination of the in vitro FPS. *Cosmetics and toiletries,* 2003, vol. 118 (10), 63-72